# EUROPEAN PATENT SPECIFICATION

(11) **EP 1 089 780 B1**
(45) Date of publication and mention of the grant of the patent: **03.10.2012**
(21) Application number: 99930285.4
(22) Date of filing: 15.06.1999
(51) Int. Cl.: A61M 1/36, A61F 7/12

(54) **SELECTIVE ORGAN COOLING APPARATUS**
VORRICHTUNG ZUR KÜHLUNG EINES AUSGEWÄHLTEN ORGANS
APPAREIL DE REFROIDISSEMENT D'UN ORGANE SELECTIONNE

(30) Priority: 23.06.1998 US 103342; 16.12.1998 US 215038
(43) Date of publication of application: 11.04.2001
(73) Proprietor: Innercool Therapies, Inc., San Diego, CA 92121 (US)
(72) Inventor: DOBAK, John, D., III, La Jolla, CA 92037 (US); LASHERAS, Juan, C., La Jolla, CA 92037 (US)
(74) Representative: Verweij, Petronella Danielle
(86) International application number: PCT/US1999/013516
(87) International publication number: WO 1999/066970

(56) References cited:
- WO-A-91/05528
- WO-A-96/40347
- WO-A-98/26831
- WO-A-99/37226
- US-A- 5 269 758
- US-A- 5 486 208
- US-A- 5 624 392
- US-A- 5 735 809

## Description

### BACKGROUND OF THE INVENTION

Field of the Invention - The present invention relates generally to the modification and control of the temperature of a selected body organ. More particularly, the invention relates to a method and intravascular apparatus for controlling organ temperature.

Background Information - Organs in the human body, such as the brain, kidney and heart, are maintained at a constant temperature of approximately 37° C. Hypothermia can be clinically defined as a core body temperature of 35° C or less. Hypothermia is sometimes characterized further according to its severity. A body core temperature in the range of 33° C to 35° C is described as mild hypothermia. A body temperature of 28° C to 32° C is described as moderate hypothermia. A body core temperature in the range of 24° C to 28° C is described as severe hypothermia.

Hypothermia is uniquely effective in reducing brain injury caused by a variety of neurological insults and may eventually play an important role in emergency brain resuscitation. Experimental evidence has demonstrated that cerebral cooling improves outcome after global ischemia, focal ischemia, or traumatic brain injury. For this reason, hypothermia may be induced in order to reduce the effect of certain bodily injuries to the brain as well as other organs.

Cerebral hypothermia has traditionally been accomplished through whole body cooling to create a condition of total body hypothermia in the range of 20° C to 30° C. However, the use of total body hypothermia risks certain deleterious systematic vascular effects. For example, total body hypothermia may cause severe derangement of the cardiovascular system, including low cardiac output, elevated systematic resistance, and ventricular fibrillation. Other side effects include renal failure, disseminated intravascular coagulation, and electrolyte disturbances. In addition to the undesirable side effects, total body hypothermia is difficult to administer.

Catheters have been developed which are inserted into the bloodstream of the patient in order to induce total body hypothermia. For example, U.S. Patent No. 3,425,419 to Dato describes a method and apparatus of lowering and raising the temperature of the human body. The Dato patent is directed to a method of inducing moderate hypothermia in a patient using a metallic catheter. The metallic catheter has an inner passageway through which a fluid, such as water, can be circulated. The catheter is inserted through the femoral vein and then through the inferior vena cava as far as the right atrium and the superior vena cava. The Dato catheter has an elongated cylindrical shape and is constructed from stainless steel. By way of example, Dato suggests the use of a catheter approximately 70 cm in length and approximately 6 mm in diameter. However, use of the Dato system implicates the negative effects of total body hypothermia described above.

Due to the problems associated with total body hypothermia, attempts have been made to provide more selective cooling. For example, cooling helmets or head gear have been used in an attempt to cool only the head rather than the patient's entire body. However, such methods rely on conductive heat transfer through the skull and into the brain. One drawback of using conductive heat transfer is that the process of reducing the temperature of the brain is prolonged. Also, it is difficult to precisely control the temperature of the brain when using conduction due to the temperature gradient that must be established externally in order to sufficiently lower the internal temperature. In addition, when using conduction to cool the brain, the face of the patient is also subjected to severe hypothermia, increasing discomfort and the likelihood of negative side effects. It is known that profound cooling of the face can cause similar cardiovascular side effects as total body cooling. Further, from a practical standpoint, cooling helmets and head gear are cumbersome and may make continued treatment of the patient difficult or impossible.

Selected organ hypothermia has been accomplished using extracorporeal perfusion, as detailed by Arthur E. Schwartz, M.D. et al., in Isolated Cerebral Hypothermia by Single Carotid Artery Perfusion of Extracorporeally Cooled Blood in Baboons, which appeared in Vol. 39, No. 3, NEUROSURGERY 577 (September, 1996). In this study, blood was continually withdrawn from baboons through the femoral artery. The blood was cooled by a water bath and then infused through a common carotid artery with its external branches occluded. Using this method, normal heart rhythm, systemic arterial blood pressure and arterial blood gas values were maintained during the hypothermia. This study showed that the brain could be selectively cooled to temperatures of 20° C without reducing the temperature of the entire body. However, external circulation of blood is not a practical approach for treating humans because the risk of infection, need for anticoagulation, and risk of bleeding is too great. Further, this method requires cannulation of two vessels making it more cumbersome to perform, particularly in emergency settings. Moreover, percutaneous cannulation of the carotid artery is difficult and potentially fatal due to the associated arterial wall trauma. Finally, this method would be ineffective to cool other organs, such as the kidneys, because the feeding arteries cannot be directly cannulated percutaneously.

Selective organ hypothermia has also been attempted by perfusion of a cold solution such as saline or perflourocarbons. This process is commonly used to protect.the heart during heart surgery and is referred to as cardioplegia. Perfusion of a cold solution has a number of drawbacks, including a limited time of administration due to excessive volume accumulation, cost, and inconvenience of maintaining the perfusate and lack of effectiveness due to the temperature dilution from the blood. Temperature dilution by the blood is a particular problem in high blood flow organs such as the brain.

WO 99/37226 (published late) describes a method and apparatus for performing hypothermia of a selected body organ, wherein a flexible catheter is inserted through the vascular system of a patient to place the distal tip of the catheter in an artery feeding the selected organ. A compressed refrigerant is pumped through the catheter to an expansion element near the distal tip of the catheter, where the refrigerant vaporizes and expands to cool a flexible heat transfer element in the distal tip of the catheter. The heat transfer element cools the blood flowing through the artery to cool the selected organ, distal to the tip of the catheter.

### SUMMARY

The prevent invention is specified in the claims.

The present invention involves an apparatus for controlling the temperature of a selected organ such as the brain.

The present invention provides a system, which may be used to selectively control the temperature of a chosen organ, while inducing a minimum of total body hypothermia. The apparatus, according to the invention, may include a catheter having a heat transfer element attached to a distal portion thereof. The heat transfer element allows the fluid proximate the selected organ to be cooled or heated. A turbulence-enhancing element is also attached to a distal portion of the catheter and is adapted to enhance turbulent blood flow along the heat transfer element, so as to increase the efficiency of the heat transfer.

An additional aspect of the invention involves a selective organ heat transfer device that includes a catheter capable of insertion into a selected blood vessel in the vascular system of a patient, a heat transfer element attached to a distal portion of the catheter, and a turbulence-enhancing element attached to a distal portion of the catheter and adapted to enhance turbulent blood flow along the heat transfer element.

In an embodiment of the above aspect of the invention, the heat transfer element includes the turbulence-enhancing element.

The turbulence-enhancing element includes a plurality of exterior surface irregularities, the surface irregularities being shaped and arranged to create repetitively changing directions of flow in surrounding fluid, the surface irregularities having a depth at least equal to the boundary layer thickness of flow between the heat transfer element and the feeding artery.

In a further embodiment of the above aspect of the invention, the surface irregularities comprise a helical ridge and a helical groove formed on each the heat transfer segment, and the helical ridge on each the heat transfer segment has an opposite helical twist to the helical ridges on adjacent the heat transfer segments.

In another embodiment of the above aspect of the invention, the surface irregularities comprise protrusions on the exterior surface, and the protrusions are axially staggered, and circumferentially overlapping, along the exterior surface.

In an additional embodiment of the above aspect of the invention, an inner coaxial tube is disposed within the heat transfer element, the inner coaxial tube is connected in fluid flow communication with an inner coaxial tube within the catheter, the heat transfer element comprises a plurality of heat transfer segments, the turbulence-enhancing element comprises at least one flexible joint connecting each of the heat transfer segments to adjacent heat transfer segments, the at least one flexible joint including an internal area in fluid flow communication with the inner coaxial tube and adapted to expand upon flow of a working fluid through the catheter.

In a further embodiment of the invention, the heat transfer element includes an inlet lumen and an outlet lumen, the outlet lumen is coupled to the inlet lumen so as to transfer working fluid between the two, the outlet lumen has a structure when expanded to induce turbulence in the blood or in the working fluid.

In a still further embodiment of the invention, the inlet lumen and the outlet lumen are made of a flexible material.

In an additional embodiment of the invention, the flexible material is rubber.

In another embodiment of the invention, the flexible material is latex rubber.

In a further embodiment of the invention, the outlet lumen has a structure to induce turbulence in the working fluid.

In a still further embodiment of the invention, the outlet lumen has the shape of a helix when expanded.

In an additional embodiment of the invention, the helix shape of the outlet lumen is tapered when expanded.

In another embodiment of the invention, the helix shape of the outlet lumen is segmentally tapered when expanded.

In a further embodiment of the invention, the inlet lumen decreases such that the inlet lumen is tapered when expanded.

In a still further embodiment of the invention, a radius of the outlet lumen decreases such that the outlet lumen is tapered when expanded.

In a further embodiment of the above aspect of the invention, the turbulence-enhancing element is located proximal of the heat transfer element.

In a still further embodiment of the above aspect of the invention, the turbulence-enhancing element includes an expandable micro-balloon.

In another embodiment of the above aspect of the invention, the turbulence-enhancing element includes an expandable micro-ring balloon.

In an additional embodiment of the above aspect of the invention, the turbulence-enhancing element includes multiple axially staggered and circumferentially overlapping protrusions located along the exterior surface.

In a further embodiment of the above aspect of the invention, the turbulence-enhancing element includes a temperature sensor wire helically wound around the exterior surface and having a temperature sensor in thermal contact with the heat transfer element for feedback control of the heat transfer element.

In a still further embodiment of the above aspect of the invention, the temperature sensor is a member from the group consisting of thermocouple and thermistor.

In an additional embodiment of the above aspect of the invention, the catheter includes a longitudinal axis and the turbulence-enhancing element includes a fan adapted to rotate about an axis coaxial with the longitudinal axis of the catheter.

In another embodiment of the above aspect of the invention, the fan is adapted to rotate upon fluid flow past the fan.

In an additional embodiment of the above aspect of the invention, a driving mechanism is adapted to rotate the fan.

In a further embodiment of the above aspect of the invention, the fan includes multiple blades adapted to have a low profile when the fan is at rest and expand when in motion.

Another aspect of the invention involves a selective organ heat transfer device, including a catheter capable of insertion into a selected blood vessel in the vascular system of a patient, means for transferring heat attached to a distal portion of the catheter, and means for enhancing turbulent blood flow along the heat transferring means attached to a distal portion of the catheter.

In an embodiment of the above aspect of the invention, the selective organ heat transfer device includes additional means for enhancing turbulent blood flow along the heat transferring means.

The selective organ heat transfer device may be used to perform a method for selectively controlling the temperature of a selected organ of a patient, the method including providing a catheter having a heat transfer element and a turbulence-enhancing element attached to a distal portion thereof; inserting the catheter through the vascular system of the patient to place the heat transfer element and turbulence-enhancing element in the feeding artery of the selected organ; creating turbulent blood flow around the heat transfer element with the turbulence-enhancing element; circulating fluid into the heat transfer element via an internal lumen of the catheter and via an internal lumen of the heat transfer element; circulating fluid out of the heat transfer element via an external lumen of the heat transfer element; and transferring heat between the heat transfer element and the blood in the feeding artery to selectively control the temperature of the selected organ, whereby the turbulent blood flow induced around the heat transfer element enhances the transfer of heat between the heat transfer element and blood.

The heat transfer element may include a plurality of segments of helical ridges and grooves having alternating directions of helical rotation, and the method includes the step of creating turbulent blood flow around the heat transfer element by establishing repetitively alternating directions of helical blood flow with the alternating helical rotations of the ridges and grooves.

In addition, the turbulence-enhancing element may be an expandable micro-ballon or micro-ring ballon, and the micro-ballon or micro-ring ballon enhances turbulence by changing the direction of the velocity vectors of the blood flow contacting the segments.

The turbulence-enhancing element may also include multiple axially staggered and circumferentially overlapping protrusions located along an exterior surface of the catheter, and the protrusions enhance turbulence by creating pre-turbulence of the blood flow prior to contacting the segments. The turbulence-enhancing element may also be a temperature sensor wire helically wound around the catheter, the temperature sensor wire has a temperature sensor in thermal contact with the heat transfer element, and the temperature sensor wire enhances turbulence by altering the direction of the blood flow contacting the segments and by creating pre-turbulence of the blood flow prior to contacting the segments, and the method further includes feedback controlling the temperature of the circulating fluid based on the temperature of the heating element.

Alternatively, the temperature sensor may be located distal of the heat transfer element, and the method further includes feedback controlling the temperature of the circulating fluid based on the temperature of blood downstream of the heating element.

The turbulence-enhancing element may also be a rotating fan.

Additionally, the turbulence-enhancing element may include one or more pulsating tubing sections, each including an interior area, and the method further includes circulating the fluid through each interior area so as to cause each tubing section to pulsate.

A further aspect of the invention involves a selective organ heat transfer device including a catheter for insertion in a blood vessel of a patient, a heat transfer element attached to a distal end of the catheter and having a plurality of exterior surface irregularities formed thereon, the surface irregularities being shaped and arranged to create repetitively changing directions of flow in surrounding fluid, the surface irregularities having a depth at least equal to the boundary layer thickness of flow between the heat transfer element and the blood vessel, and a turbulence-enhancing element associated with the heating element for imparting turbulence to blood prior to the blood reaching the heating element.

An additional aspect of the invention involves a catheter system having an inlet lumen and an outlet lumen structured and arranged to carry a working fluid having a temperature different from the adjacent blood. The outlet lumen is configured to induce turbulence in the adjacent fluid passing adjacent the outlet lumen.

Another aspect of the invention involves a catheter system to change the temperature of blood by heat transfer to or from a working fluid including an inlet lumen and an outlet lumen, the outlet lumen coupled to the inlet lumen so as to transfer the working fluid between the two, the outlet lumen having a structure when expanded to induce turbulence in the blood or in the working fluid.

In an embodiment of the above aspect of the invention, the inlet lumen and the outlet lumen are made of a flexible material.

In an additional embodiment of the above aspect of the invention, the flexible material is rubber.

In another embodiment of the above aspect of the invention, the flexible material is latex rubber.

In a further embodiment of the above aspect of the invention, the outlet lumen has a structure to induce turbulence in the working fluid.

In a still further embodiment of the above aspect of the invention, the outlet lumen has the shape of a helix when expanded.

In an additional embodiment of the above aspect of the invention, the outlet lumen is tapered when expanded.

In another embodiment of the above aspect of the invention, the helix shape of the outlet lumen is segmentally tapered when expanded.

In a further embodiment of the above aspect of the invention, a radius of the inlet lumen decreases such that the inlet lumen is tapered when expanded.

In an additional embodiment of the above aspect of the invention, a radius of the outlet lumen decreases such that the outlet lumen is tapered when expanded.

In another embodiment of the above aspect of the invention, a wire is disposed within one of at least the inlet lumen or the outlet lumen.

In a further embodiment of the above aspect of the invention, the thickness of the outlet lumen when expanded is less than about ½ mil.

In a still further embodiment of the above aspect of the invention, a length of the inlet lumen is between about 5 and 30 centimeters.

In an additional embodiment of the above aspect of the invention, a diameter of the helix of the outlet lumen is less than about 8 millimeters when expanded.

In another embodiment of the above aspect of the invention, the outer diameter of the helix of the outlet lumen, when expanded, is between about 2 millimeters and 8 millimeters and tapers to between about 1 millimeter and 2 millimeters.

In an additional embodiment of the above aspect of the invention, a length of a segment is between about 1 centimeter and 10 centimeters.

In a further embodiment of the above aspect of the invention, the radii of the inlet and outlet lumens when expanded are between about 0.5 millimeters and 2 millimeters.

In a still further embodiment of the above aspect of the invention, the outlet lumen further comprises at least one surface feature, the surface feature inducing turbulence in the fluid adjacent the outlet lumen.

In an additional embodiment of the above aspect of the invention, the outlet lumen further comprises at least one interior feature, the interior feature inducing turbulence in the working fluid.

In another embodiment of the above aspect of the invention, the surface feature is a series of helical turns formed in the outlet lumen.

In an additional embodiment of the above aspect of the invention, each pair of adjacent turns in the series of helical turns has opposite helicity.

In another embodiment of the above aspect of the invention, the surface feature is a helical shape formed in the outlet lumen.

In a further embodiment of the above aspect of the invention, the surface feature is a series of protrusions formed in the outlet lumen.

In a still further embodiment of the above aspect of the invention, the turbulence-inducing outlet lumen is adapted to induce turbulence when expanded within a free stream of blood flow when placed within an artery.

In an additional embodiment of the above aspect of the invention, the turbulence-inducing outlet lumen is adapted to induce a turbulence intensity when expanded within a free stream blood flow which is greater than 0.05.

In another embodiment of the above aspect of the invention, the turbulence-inducing exterior surface is adapted to induce turbulence when expanded during at least 20% of the period of the cardiac cycle when placed within an artery.

In an additional embodiment of the above aspect of the invention, the turbulence-inducing outlet lumen is adapted to induce turbulence when expanded throughout the period of the cardiac cycle when placed within an artery.

In a further embodiment of the above aspect of the invention, a coaxial supply catheter having an inner catheter lumen is coupled to the inlet lumen, and a working fluid supply is configured to dispense the working fluid and has an output coupled to the inner catheter lumen.

In a still further embodiment of the above aspect of the invention, the working fluid supply is configured to produce a pressurized working fluid at a temperature of between about -3°C and 36°C and at a pressure below about 5 atmospheres of pressure.

In an additional embodiment of the above aspect of the invention, the turbulence-inducing outlet lumen includes a surface coating or treatment to inhibit clot formation.

In another embodiment of the above aspect of the invention, the surface coating or treatment includes heparin.

In a further embodiment of the above aspect of the invention, a stent is coupled to the distal end of the inlet lumen.

An additional aspect of the present invention involves a catheter system to change the temperature of blood by heat transfer to or from a working fluid including an inlet lumen and an outlet lumen, the output lumen tapered when expanded from a first radius to a second radius, the outlet lumen coupled to the inlet lumen so as to transfer the working fluid between the two, such that the taper of the outlet lumen allows the outlet lumen to be placed in an artery having a radius less than the first radius.

Another aspect of the invention involves a catheter system to change the temperature of blood by heat transfer to or from a working fluid including an inlet lumen and an outlet lumen, the outlet lumen segmentally tapered when expanded from a first radius to a second radius, the outlet lumen coupled to the inlet lumen so as to transfer the working fluid between the two, such that adjacent segments of the outlet lumen are separated by joints, the joints having a radius less than that of either adjacent segment, and such that the taper of the outlet lumen when expanded allows the outlet lumen to be placed in an artery having a radius less than the first radius.

A further aspect of the invention involves a catheter system to cool the temperature of blood by heat transfer to a working fluid including a substantially straight inlet lumen to deliver a working fluid, and an outlet lumen that when expanded substantially helically surrounds the inlet lumen to remove the working fluid, the helical shape of the outlet lumen to induce turbulence in the working fluid or in the blood or both, such that the helical shape of the outlet lumen is sufficient to induce a turbulence intensity in the blood of greater than about 0.05.

A still further aspect of the invention involves a catheter system to cool the temperature of blood by heat transfer to a working fluid, including a segmentally tapered and substantially straight inlet lumen to deliver a working fluid, and an outlet lumen, the outlet lumen segmentally tapered when expanded and helically surrounding the inlet lumen to remove the working fluid, the helical and segmentally tapered shape of the outlet lumen to induce turbulence in the working fluid and in the blood, such that the shape of the outlet lumen is sufficient to induce a turbulence intensity in the blood of greater than about 0.05.

An additional aspect of the present invention involves a medical catheter system to cool the temperature of blood by heat transfer to a working fluid including a substantially straight expandable inlet lumen to deliver a working fluid, and a tapered expandable outlet lumen substantially surrounding the inlet lumen to remove the working fluid, an external surface of the outlet lumen having surface features to induce turbulence in the working fluid and in the blood, such that the surface features are sufficient to induce a turbulence intensity in the blood of greater than about 0.05.

Another aspect of the present invention involves a medical catheter system to cool the temperature of blood by heat transfer to a working fluid including a substantially straight expandable inlet lumen to deliver a working fluid, and a tapered expandable outlet lumen substantially surrounding the inlet lumen to remove the working fluid, an external surface of the outlet lumen having a spiral formed thereon to induce turbulence in the working fluid and in the blood, such that the spiral surface feature is sufficient to induce a turbulence intensity in the blood of greater than about 0.05.

A further aspect of the present invention involves a medical catheter system to cool the temperature of blood by heat transfer to a working fluid including a substantially straight expandable inlet lumen to deliver a working fluid, and a tapered expandable outlet lumen substantially surrounding the inlet lumen to remove the working fluid, an external surface of the outlet lumen having staggered protrusions formed thereon to induce turbulence in the working fluid and in the blood, such that the staggered protrusions are sufficient to induce a turbulence intensity in the blood of greater than about 0.05.

The selective organ heat transfer device may be used to perform a catheter method of changing the temperature of blood by heat transfer including inserting an expandable heat transfer element into an artery or vein; expanding the expandable heat transfer element by delivering a working fluid to the expandable heat transfer element, the temperature of the working fluid different from that of the blood; and inducing turbulence in the working fluid by passing the working fluid through a turbulence-inducing path, such that turbulence is induced in a substantial portion of a free stream of blood.

The selective organ heat transfer device may also be used to perform a method of changing the temperature of blood by heat transfer including inserting an expandable heat transfer element into a flow of blood in an artery or vein; and expanding the expandable heat transfer element by delivering a working fluid having a temperature different than the blood temperature to the expandable heat transfer element, the expandable heat transfer element having a turbulence-inducing structure when expanded, such that turbulence is induced in a substantial portion of a free stream of blood.

The method may further include delivering the working fluid at a temperature of between about -3°C and 36°C.

In an additional embodiment of the above aspect of the invention, the method further includes delivering the working fluid at a pressure of less than about 5 atmospheres.

The method may further include delivering the working fluid at a pressure of less than about 5 atmospheres, or delivering the working fluid at a pressure of between about 1 and 5 atmospheres.

The method may also include absorbing more than about 75 watts of heat from the blood, and/or inducing blood turbulence with a turbulence intensity greater than about 0.05 within the carotid artery.

Also,expanding may further comprise passing the working fluid through a helical-shaped structure. In addition, the method may further include inducing blood turbulence from between 20% and 100% of the period of the cardiac cycle within the carotid artery.

The selective organ heat transfer device may also be used to perform a method for selectively cooling an organ in a body of a patient including introducing a catheter having an expandable heat transfer element into a blood vessel supplying the organ, expanding the heat transfer element, and inducing free stream turbulence in blood flowing over the heat transfer element, such that heat is removed from the blood to cool the organ without substantially cooling the entire body.

Another aspect of the present invention involves a catheter system to change the temperature of an adjacent material by heat transfer to or from a working fluid including an inlet lumen and an outlet lumen, the outlet lumen coupled to the inlet lumen so as to transfer the working fluid between the two, the outlet lumen having a structure to induce turbulence in the working fluid.

Other advantages, features, and objects of the invention are described in the detailed description below.

### BRIEF DESCRIPTION OF THE DRAWINGS

Figure 1 is a graph illustrating the velocity of steady state turbulent flow as a function of time;
Figure 2A is a graph showing the velocity of the blood flow within an artery as a function of time;
Figure 2B is a graph illustrating the velocity of steady state turbulent flow under pulsatile conditions as a function of time, similar to arterial blood flow;
Figure 2C is an elevation view of a turbulence inducing heat transfer element constructed in accordance with an embodiment of the invention within an artery;
Figure 3A is a velocity profile diagram showing a typical steady state Poiseuillean flow driven by a constant pressure gradient;
Figure 3B is a velocity profile diagram showing blood flow velocity within an artery, averaged over the duration of the cardiac pulse;
Figure 3C is a velocity profile diagram showing blood flow velocity within an artery, averaged over the duration of the cardiac pulse, after insertion of a smooth heat transfer element within the artery;
Figure 4 is an elevation view of one embodiment of a heat transfer element according to the invention;
Figure 5 is longitudinal section view of the heat transfer element of Figure 4 taken along line 5-5;
Figure 6 is a transverse section view of the heat transfer element of Figure 4 taken along line 6-6;
Figure 7 is a perspective view of the heat transfer element of Figure 4 in use within a blood vessel;
Figure 8 is a cut-away perspective view of an alternative embodiment of a heat transfer element according to the invention;
Figure 9 is a transverse section view of the heat transfer element of Figure 8;
Figure 10 is an elevation view of an additional embodiment of a heat transfer mechanism according to the invention in use within a blood vessel;
Figure 11 is a longitudinal cross-sectional view of the heat transfer mechanism illustrated in Figure 10 taken along line 11-11;
Figure 12 is an elevation view of another embodiment of a heat transfer mechanism according to the invention in use within a blood vessel;
Figure 13 is an elevation view of an additional embodiment of a heat transfer mechanism according to the invention in use within a blood vessel;
Figure 14 is an elevation view of a further embodiment of a heat transfer mechanism according to the invention in use within a blood vessel;
Figure 15 is a perspective view of a heat transfer mechanism constructed in accordance with a still further embodiment of the invention in use within a blood vessel;
Figures 16A, 16B are transverse section views taken along lines 16A-16A, 16B-16B of Figure 15, with Figure 16A illustrating the heat transfer mechanism in a low-profile position and Figure 16B illustrating the heat transfer mechanism in an expanded position;
Figure 17 is a cross-sectional view, similar to Figures 16A and 16B, of an additional embodiment of a heat transfer mechanism according to the invention;
Figures 18A, 18B, 18C are elevation views of a further embodiment of a heat transfer mechanism according to the invention in use within a blood vessel during various states of operation;
Figure 19 is a side schematic view of an expandable turbulence-inducing heat transfer element according to an additional embodiment of the invention, as the same is disposed within an artery;
Figure 20 illustrates an expandable turbulence-inducing heat transfer element according to an alternative embodiment of the invention employing a surface area enhancing taper and a turbulence-inducing shape;
Figure 21 illustrates a tapered joint which may be employed in the embodiment of Fig. 20;
Figure 22 illustrates a turbulence-inducing heat transfer element according to a further embodiment of the invention employing a surface area enhancing taper and turbulence-inducing surface features;
Figure 23 illustrates a type of turbulence-inducing surface feature which may be employed in the heat transfer element of the embodiment of Fig. 22. In Fig. 23 a spiral feature is shown;
Figure 24 illustrates a heat transfer element according to a still further embodiment of the invention employing a surface area enhancing taper; and
Figure 25 is a schematic representation of an embodiment of the invention being used to cool the brain of a patient.

### DETAILED DESCRIPTION OF THE INVENTION

In order to intravascularly regulate the temperature of a selected organ, a heat transfer element may be placed in the feeding artery of the organ to absorb or deliver the heat from or to the blood flowing into the organ. The transfer of heat may cause either a cooling or a heating of the selected organ. The heat transfer element should be small enough to fit within the feeding artery while still allowing sufficient blood flow to reach the organ in order to avoid ischemic organ damage. The heat transfer element should also provide the necessary heat transfer rate to produce the desired cooling or heating effect within the organ. By placing the heat transfer element within the feeding artery of an organ, the temperature of an organ can be controlled without significantly affecting the remaining parts of the body. These points can be illustrated by using brain cooling as an example.

The common carotid artery supplies blood to the head and brain. The internal carotid artery branches off of the common carotid to directly supply blood to the brain. To selectively cool the brain, a heat transfer element may be placed into the common carotid artery, the internal carotid artery, or both. The internal diameter of the common carotid artery ranges from 6 to 8 mm and the length ranges from 80 to 120 mm. Thus, the heat transfer element residing in one of these arteries should not be much larger than 4 mm in diameter in order to avoid occluding the vessel.

It is advantageous that the heat transfer element be flexible in order to be placed within a small feeding artery of an organ. Feeding arteries, like the carotid artery, branch off the aorta at various levels. Subsidiary arteries continue to branch off the initial branches. For example, the internal carotid artery is a small diameter artery that branches off of the common carotid artery near the angle of the jaw. Because the heat transfer element is typically inserted into a peripheral artery, such as the femoral artery, and accesses the feeding artery by initially passing though a series of one or more of these branches, the flexibility of the heat transfer element is highly advantageous. Further, the heat transfer element is ideally constructed from a highly thermally conductive material, such as metal, in order to facilitate heat transfer. The use of a highly thermally conductive material increases the heat transfer rate for a given temperature differential between the heat transfer substance (e.g. coolant) within the heat transfer element and the blood. This facilitates the use of a higher temperature coolant within the heat transfer element, allowing safer coolants, such as water, to be used. Highly thermally conductive materials, such as metals, tend to be rigid. Therefore, where more rigid materials such as metals are used, the design of the heat transfer element should facilitate flexibility in an inherently inflexible material.

In order to obtain the benefits of hypothermia described above, it is desirable to reduce the temperature of the blood flowing to the brain to between 30° C and 32° C. Given that a typical brain has a blood flow rate through each carotid artery (right and left) of approximately 250-375 cubic centimeters per minute, it is desirable for the heat transfer element to absorb 75-175 Watts of heat when placed in one of the carotid arteries, to induce the desired cooling effect. It should be noted that smaller organs may have less blood flow in the supply artery and may require less heat transfer, such as 25 Watts.

When a heat transfer element is inserted coaxially into an artery, the primary mechanism of heat transfer between the surface of the heat transfer element and the blood is forced convection. Convection relies upon the movement of fluid to transfer heat. Forced convection results when an external force causes motion within the fluid. In the case of arterial flow, the beating heart causes the motion of the blood around the heat transfer element.

The magnitude of the heat transfer rate is proportional to the surface area of the heat transfer element, the temperature differential, and the heat transfer coefficient of the heat transfer element.

As noted above, the receiving artery into which the heat transfer element is placed has a limited diameter and length. Thus, surface area of the heat transfer element must be limited to avoid significant obstruction of the artery, and to allow the heat transfer element to easily pass through the vascular system. For placement within the internal and common carotid artery, the cross sectional diameter of the heat transfer element is limited to about 4 mm, and its length is limited to approximately 10 cm.

The temperature differential can be increased, in the case of cooling, by decreasing the surface temperature of the heat transfer element. However, the minimum allowable surface temperature is limited by the characteristics of blood. Blood freezes at approximately 0°C. When the blood approaches freezing, ice emboli may form in the blood which may lodge downstream, causing serious ischemic injury. Furthermore, reducing the temperature of the blood also increases its viscosity, which results in a small decrease in the value of the convection heat transfer coefficient. In addition, increased viscosity of the blood may result in an increase in the pressure drop within the artery, thus compromising the flow of blood to the brain. Given the above constraints, it is advantageous to limit the minimum allowable surface temperature of the heat transfer element to approximately 5° C. This results in a maximum temperature differential between the blood stream and the heat transfer element of approximately 32° C, where the patient has a normal 37° C temperature.

The mechanisms by which the value of the convection heat transfer coefficient may be increased are complex. However, it is well known that the convection heat transfer coefficient increases with the level of turbulent kinetic energy in the fluid flow. Thus, it is advantageous to have turbulent blood flow in contact with the heat transfer element.

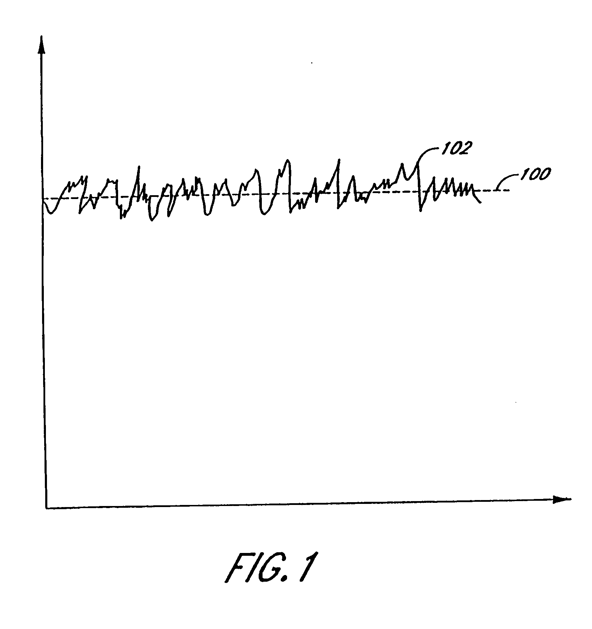

Figure 1 is a graph illustrating steady state turbulent flow. The vertical axis is the velocity of the flow. The horizontal axis represents time. The average velocity of the turbulent flow is shown by a line 100. The actual instantaneous velocity of the flow is shown by a curve 102.

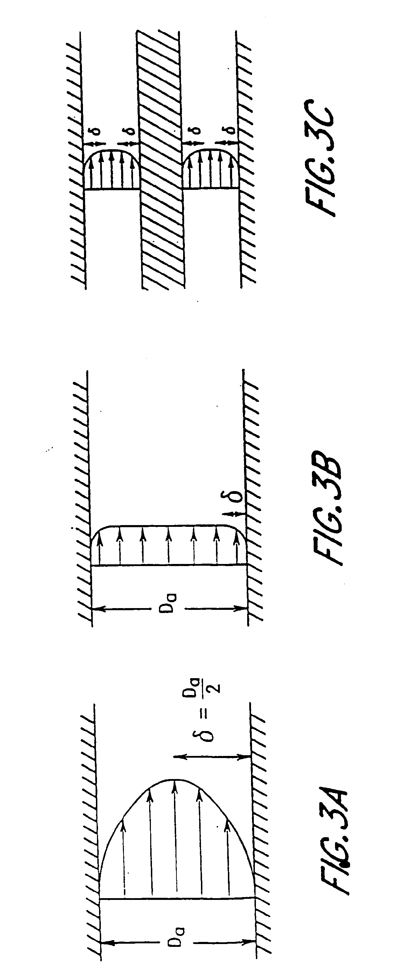

Under constant pressure conditions, the flow in a pipe is Poiseuillean. Figure 3A is a velocity profile diagram showing a typical steady state Poiseuillean flow driven by constant pressure. The velocity of the fluid across the pipe is shown in Figure 3A by the parabolic curve and corresponding velocity vectors. The velocity of the fluid in contact with the wall of the pipe is zero. The boundary layer is the region of the flow in contact with the pipe surface in which viscous stresses are dominant. In steady state Poiseuillean flow, the boundary layer develops until it reaches the pipe center line. For example, the boundary layer thickness in Figure 3A is one half of the diameter of the pipe.

Under conditions of Poiseuillean flow, the Reynolds number (i.e. the ratio of inertial forces to viscous forces) can be used to characterize the level of turbulent kinetic energy. For Poiseuillean flows, Reynolds numbers must be greater than about 2300 to cause a laminar to turbulent transition. Further, when the Reynolds number is greater than about 2000, the boundary layer is receptive to "tripping". Tripping is a process by which a small perturbation in the boundary layer can create turbulent conditions. The receptivity of a boundary layer to "tripping" is proportional to the Reynolds number and is nearly zero for Reynolds numbers less than 2000.

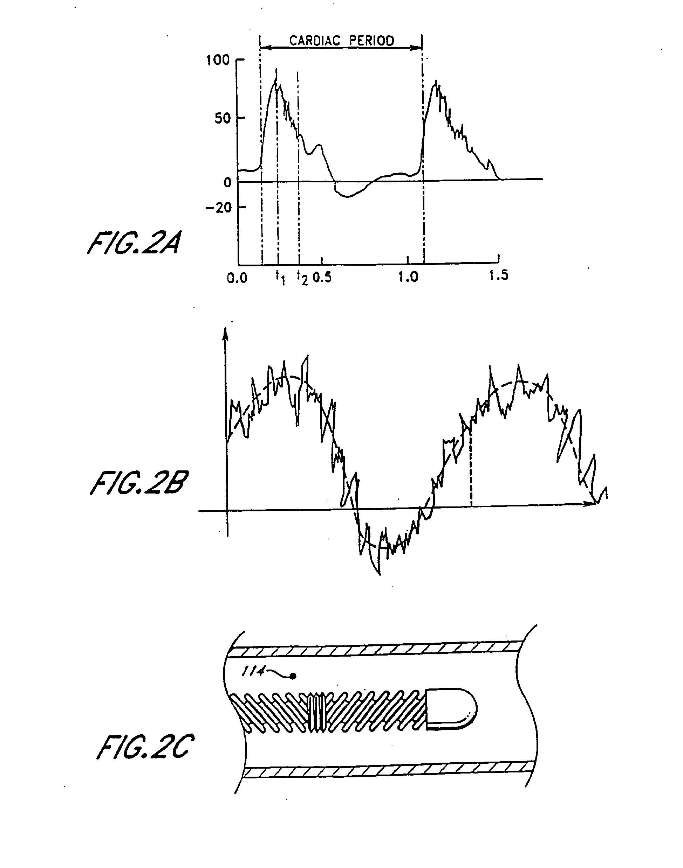

Blood flow in arteries is induced by the beating heart and is therefore pulsatile, complicating the fluid mechanics analysis. Figure 2A is a graph showing the velocity of the blood flow within an artery as a function of time. The beating heart provides pulsatile flow with an approximate period of 0.5 to 1 second. This is known as the period of the cardiac cycle. The horizontal axis in Figure 2A represents time in seconds and the vertical axis represents the average velocity of blood in centimeters per second. Although very high velocities are reached at the peak of the pulse, the high velocity occurs for only a small portion of the cycle. In fact, as shown in figure 2A, the velocity of the blood reaches zero in the carotid artery at the end of a pulse and temporarily reverses.

Because of the relatively short duration of the cardiac pulse, the blood flow in the arteries does not develop into classic Poiseuillean flow. Figure 3B is a velocity profile diagram showing blood flow velocity within an artery averaged over the cardiac pulse. The majority of the flow within the artery has the same velocity. The boundary layer where the flow velocity decays from the free stream value to zero is very thin, typically 1/6 to 1/20 of the diameter of the artery, as opposed to one half of the diameter of the artery in the Poiseuillean flow condition.

As noted above, if the flow in the artery were steady rather than pulsatile, the transition from laminar to turbulent flow would occur when the value of the Reynolds number exceeds about 2000. However, in the pulsatile arterial flow, the value of the Reynolds number varies during the cardiac cycle, just as the flow velocity varies. In pulsatile flows, due to the enhanced stability associated with the acceleration of the free stream flow, the critical value of the Reynolds number at which the unstable modes of motion grow into turbulence is found to be much higher, perhaps as high as 9000.

The blood flow in the arteries of interest remains laminar over more than 80% of the cardiac cycle. Referring again to Figure 2A, the blood flow is turbulent from approximately time t₁ until time t₂ during a small portion of the descending systolic flow, which is less than 20% of the period of the cardiac cycle. If a heat transfer element is placed inside the artery, heat transfer will be facilitated during this short interval. However, to transfer the necessary heat to cool the brain, turbulent kinetic energy should be produced and sustained throughout the entire period of the cardiac cycle.

A thin boundary layer has been shown to form during the cardiac cycle. This boundary layer will form over the surface of a smooth heat transfer element. Figure 3C is a velocity profile diagram showing blood flow velocity within an artery, averaged over the cardiac pulse, after insertion of a smooth heat transfer element within the artery. In Figure 3C, the diameter of the heat transfer element is about one half of the diameter of the artery. Boundary layers develop adjacent to the heat transfer element as well as next to the walls of the artery. Each of these boundary layers has approximately the same thickness as the boundary layer which would have developed at the wall of the artery in the absence of the heat transfer element. The free stream flow region is developed in an annular ring around the heat transfer element.

One way to increase the heat transfer rate is to create a turbulent boundary layer on the heat transfer element surface. However, turbulence in the very thin boundary layer will not produce sufficient kinetic energy to produce the necessary heat transfer rate. Therefore, to induce sufficient turbulent kinetic energy to increase the heat transfer rate sufficiently to cool the brain, a stirring mechanism, which abruptly changes the direction of velocity vectors, may be utilized. This can create high levels of turbulence intensity in the free stream, thereby sufficiently increasing the heat transfer rate.

This turbulence intensity should ideally be sustained for a significant portion of the cardiac cycle. Further, turbulent kinetic energy should ideally be created throughout the free stream and not just in the boundary layer. Figure 2B is a graph illustrating the velocity of continually turbulent flow under pulsatile conditions as a function of time, which would result in optimal heat transfer in arterial blood flow. Turbulent velocity fluctuations are seen throughout the cycle as opposed to the short interval of fluctuations seen in Figure 2A between time t₁ and time t₂. These velocity fluctuations are found within the free stream. The turbulence intensity shown in Figure 2B is at least 0.05. In other words, the instantaneous velocity fluctuations deviate from the mean velocity by at least 5%. Although, ideally, turbulence is created throughout the entire period of the cardiac cycle, the benefits of turbulence are obtained if the turbulence is sustained for 75%, 50% or even as low as 30% or 20% of the cardiac cycle.

To create the desired level of turbulence intensity in the blood free stream during the whole cardiac cycle, one embodiment of the invention uses a modular design. This design creates helical blood flow and produces a high level of turbulence in the free stream by periodically forcing abrupt changes in the direction of the helical blood flow. Figure 2C is a perspective view of such a turbulence inducing heat transfer element within an artery. Turbulent flow would be found at point 114, in the free stream area. The abrupt changes in flow direction are achieved through the use of a series of two or more heat transfer segments, each comprised of one or more helical ridges. To affect the free stream, the depth of the helical ridge is larger than the thickness of the boundary layer which would develop if the heat transfer element had a smooth cylindrical surface.

The use of periodic abrupt changes in the helical direction of the blood flow in order to induce strong free stream turbulence may be illustrated with reference to a common clothes washing machine. The rotor of a washing machine spins initially in one direction causing laminar flow. When the rotor abruptly reverses direction, significant turbulent kinetic energy is created within the entire wash basin as the changing currents cause random turbulent motion within the clothes-water slurry.

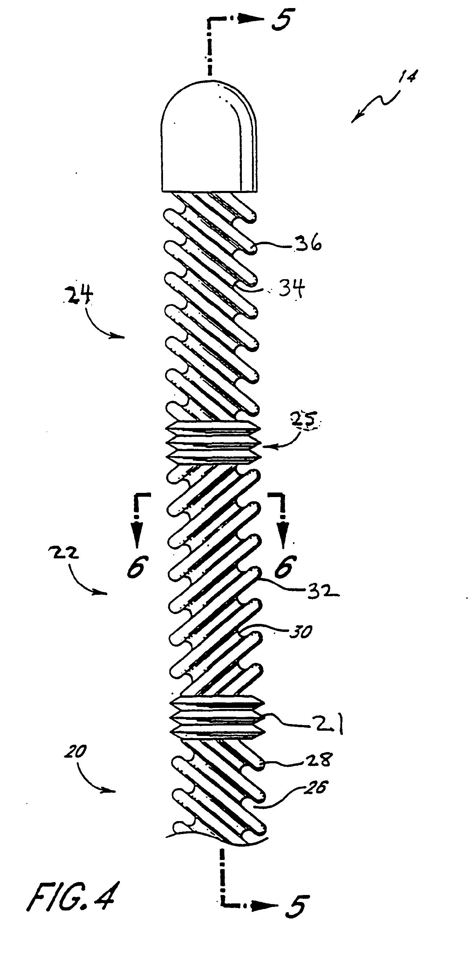

Figure 4 is an elevation view of one embodiment of a heat transfer element 14 according to the present invention. The heat transfer element 14 is comprised of a series of elongated, articulated heat transfer segments 20, 22, 24 and bellows 21 and 25. Three such segments are shown in this embodiment, but two or more such segments could be used instead. As seen in Figure 4, a first elongated heat transfer segment 20 is located at the proximal end of the heat transfer element 14. A turbulence-inducing exterior surface of the segment 20 comprises four parallel helical ridges 28 with four parallel helical grooves 26 therebetween. One, two, three, or more parallel helical ridges 28 could also be used and are within the scope of the present invention. In this embodiment, the helical ridges 28 and the helical grooves 26 of the heat transfer segment 20 have a left hand twist, referred to herein as a counter-clockwise spiral or helical rotation, as they proceed toward the distal end of the heat transfer segment 20.

The first heat transfer segment 20 is coupled to a second elongated heat transfer segment 22 by a first bellows section 21, which provides flexibility and compressibility. The second heat transfer segment 22 comprises one or more helical ridges 32 with one or more helical grooves 30 therebetween. The ridges 32 and grooves 30 have a right hand, or clockwise, twist as they proceed toward the distal end of the heat transfer segment 22. The second heat transfer segment 22 is coupled to a third elongated heat transfer segment 24 by a second bellows section 25. The third heat transfer segment 24 comprises one or more helical ridges 36 with one or more helical grooves 34 therebetween. The helical ridge 36 and the helical groove 34 have a left hand, or counter-clockwise, twist as they proceed toward the distal end of the heat transfer segment 24. Thus, successive heat transfer segments 20, 22, 24 of the heat transfer element 14 alternate between having clockwise and counterclockwise helical twists. The actual left or right hand twist of any particular segment is immaterial, as long as adjacent segments have opposite helical twist.

In addition, the rounded contours of the ridges 28, 32, 36 also allow the heat transfer element 14 to maintain a relatively atraumatic profile, thereby minimizing the possibility of damage to the blood vessel wall. A heat transfer element according to the present invention may be comprised of two, three, or more heat transfer segments.

The bellows sections 21, 25 are formed from seamless and nonporous materials, such as metal, and therefore are impermeable to gas, which can be particularly important, depending on the type of working fluid which is cycled through the heat transfer element 14. The structure of the bellows sections 21, 25 allows them to bend, extend and compress, which increases the flexibility of the heat transfer element 14 so that it is more readily able to navigate through blood vessels. The bellows sections 21, 25 also provide for axial compression of the heat transfer element 14, which can limit the trauma when the distal end of the heat transfer element 14 abuts a blood vessel wall. The bellows sections 21, 25 are also able to tolerate cryogenic temperatures without a loss of performance.

The exterior surfaces of the heat transfer element 14 can be made from metal, and may comprise very high thermally conductive material such as nickel, thereby, facilitating heat transfer. Alternatively, other metals such as stainless steel, titanium, aluminum, silver, copper and the like, can be used, with or without an appropriate coating or treatment to enhance biocompatibility or inhibit clot formation. Suitable biocompatible coatings include, e.g., gold, platinum or polymer paralyene. The heat transfer element 14 may be manufactured by plating a thin layer of metal on a mandrel that has the appropriate pattern. In this way, the heat transfer element 14 may be manufactured inexpensively in large quantities, which is an important feature for a disposable medical device.

Because the heat transfer element 14 may dwell within the blood vessel for extended periods of time, such as 24-48 hours or even longer, it may be desirable to treat the surfaces of the heat transfer element 14 to avoid clot formation. In particular, one may wish to treat the bellows sections 21, 25 because stagnation of the blood flow may occur in the convolutions, thus, allowing clots to form and cling to the surface to form a thrombus. One means by which to prevent thrombus formation is to bind an antithrombogenic agent to the surface of the heat transfer element 14. For example, heparin is known to inhibit clot formation and is also known to be useful as a biocoating. Alternatively, the surfaces of the heat transfer element 14 may be bombarded with ions such as nitrogen. Bombardment with nitrogen can harden and smooth the surface and, thus, prevent adherence of clotting factors to the surface.

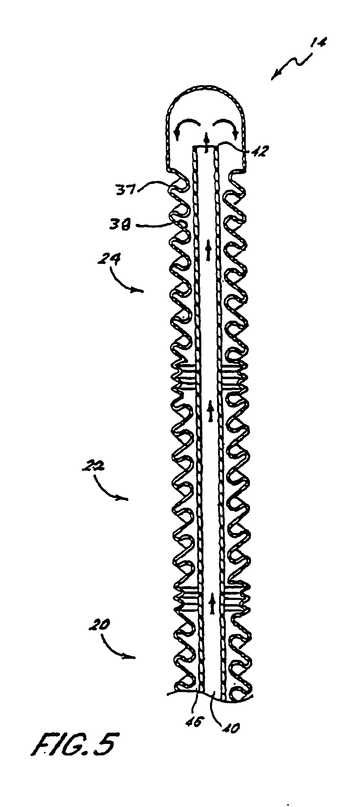

Figure 5 is a longitudinal sectional view of the heat transfer element 14 of the invention, taken along line 5-5 in Figure 4. An inner tube 40 creates an inner coaxial lumen 40 and an outer coaxial lumen 46 within the heat transfer element 14. Once the heat transfer element 14 is in place in the blood vessel, a working fluid such as saline or other aqueous solution may be circulated through the heat transfer element 14. Fluid flows from a working fluid chiller and pump, up a supply catheter and into the inner coaxial lumen 40. At the distal end of the heat transfer element 14, the working fluid exits the inner coaxial lumen 40 and enters the outer lumen 46. As the working fluid flows through the outer lumen 46, heat is transferred from the working fluid to the exterior surface 37 of the heat transfer element 14. Because the heat transfer element 14 is constructed from highly conductive material, the temperature of its exterior surface 37 may reach very close to the temperature of the working fluid. The tube 42 may be formed as an insulating divider, to thermally separate the inner lumen 40 from the outer lumen 46. For example, insulation may be achieved by creating longitudinal air channels in the wall of the insulating tube 42. Alternatively, the insulating tube 42 may be constructed of a non-thermally conductive material like polytetrafluoroethylene or some other polymer.

It is important to note that the same mechanisms that govern the heat transfer rate between the exterior surface 37 of the heat transfer element 14 and the blood also govern the heat transfer rate between the working fluid and the interior surface 38 of the heat transfer element 14. The heat transfer characteristics of the interior surface 38 is particularly important when using water, saline or some other fluid which remains a liquid, as the coolant. Other coolants such as freon undergo nucleate boiling and create turbulence through a different mechanism. Saline is a safe coolant because it is non-toxic, and leakage of saline does not result in a gas embolism, which could occur with the use of boiling refrigerants. Since turbulence in the coolant is enhanced by the shape of the interior surface 38 of the heat transfer element 14, the coolant can be delivered to the heat transfer element 14 at a warmer temperature, compared to coolant delivered along a smooth interior surface, and still achieve the necessary heat transfer rate.

This has a number of beneficial implications in the need for insulation along the catheter shaft length. Due to the decreased need for insulation, the catheter shaft diameter can be made smaller. The enhanced heat transfer characteristics of the interior surface of the heat transfer element 14 also allow the working fluid to be delivered to the heat transfer element 14 at lower flow rates and lower pressures. High pressures may make the heat transfer element stiff and cause it to push against the wall of the blood vessel, thereby shielding part of the exterior surface 37 of the heat transfer element 14 from the blood. Because of the increased heat transfer characteristics achieved by the alternating helical ridges 28, 32, 36, the pressure of the working fluid may be as low as 5 atmospheres, 3 atmospheres, 2 atmospheres or even less than 1 atmosphere.

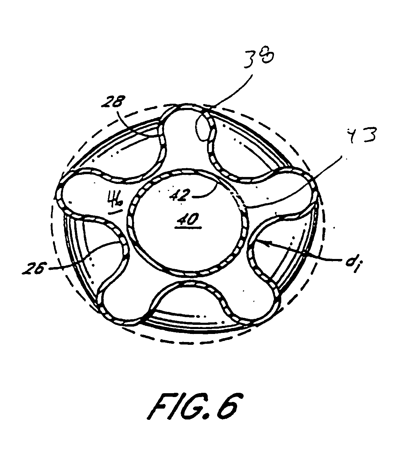

Figure 6 is a transverse sectional view of the heat transfer element 14 of the invention, taken along the line 6-6 in Figure 4. In Figure 6, the coaxial construction of the heat transfer element 14 is clearly shown. The inner coaxial lumen 40 is defined by the insulating coaxial tube 42. The outer lumen 46 is defined by the exterior surface 43 of the insulating coaxial tube 42 and the interior surface 38 of the heat transfer element 14. In addition, the helical ridges 28 and helical grooves 26 may be seen in Figure 6. As noted above, in the preferred embodiment, the depth of the grooves, dᵢ, is greater than the boundary layer thickness which would have developed if a cylindrical heat transfer element were introduced. For example, in a heat transfer element 14 with a 4 mm outer diameter, the depth of the grooves, d_{i,} may be approximately equal to 1 mm if designed for use in the carotid artery. Although Figure 6 shows four ridges and four grooves, the number of ridges and grooves may vary. Thus, heat transfer elements with 1, 2, 3, 4, 5, 6, 7, 8 or more ridges are specifically contemplated.

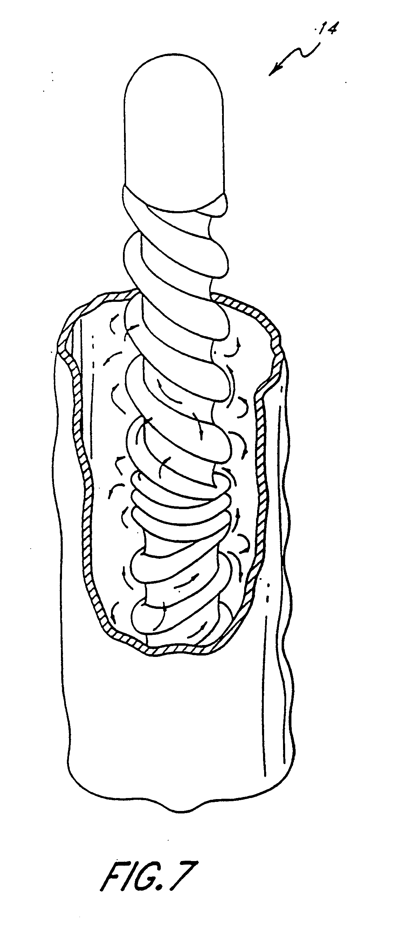

Figure 7 is a perspective view of the heat transfer element 14 in use within a blood vessel. Beginning from the proximal end of the heat transfer element (not shown in Figure 7), as the blood moves forward during the systolic pulse, the first helical heat transfer segment 20 induces a counter-clockwise rotational inertia to the blood. As the blood reaches the second segment 22, the rotational direction of the inertia is reversed, causing turbulence within the blood. Further, as the blood reaches the third segment 24, the rotational direction of the inertia is again reversed. The sudden changes in flow direction actively reorient and randomize the velocity vectors, thus, ensuring turbulence throughout the bloodstream. During turbulent flow, the velocity vectors of the blood become more random and, in some cases, become perpendicular to the axis of the artery. In addition, as the velocity of the blood within the artery decreases and reverses direction during the cardiac cycle, additional turbulence is induced and turbulent motion is sustained throughout the duration of each pulse through the same mechanisms described above.

Thus, a large portion of the volume of warm blood in the vessel is actively brought in contact with the heat transfer element 14, where it can be cooled by direct contact, rather than being cooled largely by conduction through adjacent laminar layers of blood. As noted above, the depth of the grooves 26, 30, 34 is greater than the depth of the boundary layer which would develop if a straight-walled heat transfer element were introduced into the blood stream. In this way, free stream turbulence is induced. In the preferred embodiment, in order to create the desired level of turbulence in the entire blood stream during the whole cardiac cycle, the heat transfer element 14 creates a turbulence intensity greater than 0.05. The turbulence intensity may be greater than 0.055, 0.06, 0.07 or up to 0.10 or 0.20 or greater. If the heat transfer element according to the invention were placed in a pipe approximately the same size as an artery carrying a fluid having a similar velocity, density and viscosity of blood and having a constant (rather than pulsatile) flow, Reynolds numbers of greater than 1,900, 2,000, 2,100, 2,200 or even as much as 2,300, 2,400 or 2,600 or greater would be developed. Further, the design shown in Figures 4, 5, 6 and 7 provides a similar mixing action for the working fluid inside the heat transfer element 14.

The heat transfer element 14 has been designed to address all of the design criteria discussed above. First, the heat transfer element 14 is flexible and is made of highly conductive material. The flexibility is provided by a segmental distribution of bellows sections 21, 25 which provide an articulating mechanism. Bellows have a known convoluted design which provides flexibility. Second, the exterior surface area 37 has been increased through the use of helical ridges 28, 32, 36 and helical grooves 26, 30, 34. The ridges also allow the heat transfer element 14 to maintain a relatively atraumatic profile, thereby minimizing the possibility of damage to the vessel wall. Third, the heat transfer element 14 has been designed to promote turbulent kinetic energy both internally and externally. The segment design allows the direction of the grooves to be reversed between segments. The alternating helical rotations create an alternating flow that results in mixing the blood in a manner analogous to the mixing action created by the rotor of a washing machine that switches directions back and forth. This mixing action is intended to promote high level turbulent kinetic energy to enhance the heat transfer rate. The alternating helical design also causes beneficial mixing, or turbulent kinetic energy, of the working fluid flowing internally.

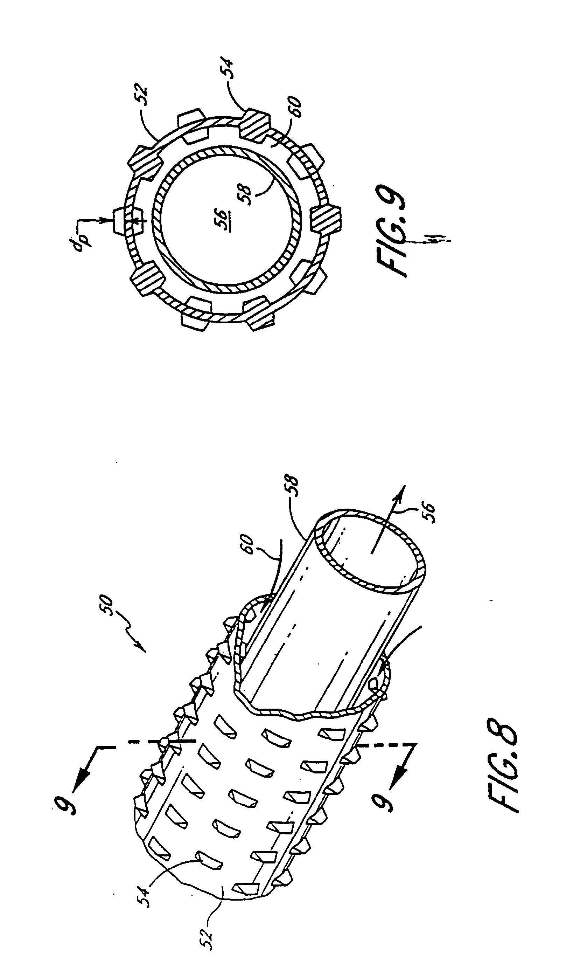

Figure 8 is a cut-away perspective view of an alternative embodiment of a heat transfer element 50. An external surface 52 of the heat transfer element 50 is covered with a series of axially staggered, circumferentially overlapping protrusions 54. The staggered, overlapping nature of the protrusions 54 is readily seen with reference to Figure 9 which is a transverse cross-sectional view taken along the line 9-9 in Figure 8. In order to induce free stream turbulence, the height, dₚ, of the staggered protrusions 54 is greater than the thickness of the boundary layer which would develop if a smooth heat transfer element had been introduced into the blood stream. As the blood flows along the external surface 52, it collides with one of the staggered protrusions 54 and turbulent flow is created. As the blood divides and swirls along side of the first staggered protrusion 54, it collides with another staggered protrusion 54 within its path preventing the relamination of the flow and creating yet more turbulence. In this way, the velocity vectors are randomized and free stream turbulence is created. Of course, other surface features may also be used which result in turbulence in fluids flowing past them. These include spirals, helices, protrusions, various polygonal bodies, pyramids, tetrahedrons, wedges, etc.

As is the case with the preferred embodiment, this geometry also induces a turbulent effect on the internal coolant flow. A working fluid is circulated up through an inner coaxial lumen 56 defined by an insulating coaxial tube 58 to a distal tip of the heat transfer element 50. The working fluid then traverses an outer coaxial lumen 60 in order to transfer heat to the exterior surface 52 of the heat transfer element 50. The inside surface of the heat transfer element 50 is similar to the exterior surface 52, in order to induce turbulent flow of the working fluid.

The embodiment of Figures 8 and 9 may result in a Nusselt number ("Nu") of about 1 to 50. The Nusselt number is the ratio of the heat transfer rate with fluid flow to the heat transfer rate in the absence of fluid flow N = Q_{flow} / Q_{no-flow} = h̅_{c} / (k/δ). The magnitude of the enhancement in heat transfer by fluid flow can be estimated by the Nusselt number. For convective heat transfer between blood and the surface of the heat transfer element, Nusselt numbers of 30-80 have been found to be appropriate for selective cooling applications of various organs in the human body. Nusselt numbers are generally dependent on several other numbers: the Reynolds number, the Womersley number, and the Prandtl number.

In an alternative embodiment of the invention, one or more of the segments 20, 22, 24, described with respect to Figures 4-7, may be replaced with a segment having overlapping protrusions 54, such as those described with respect to Figures 8-9.

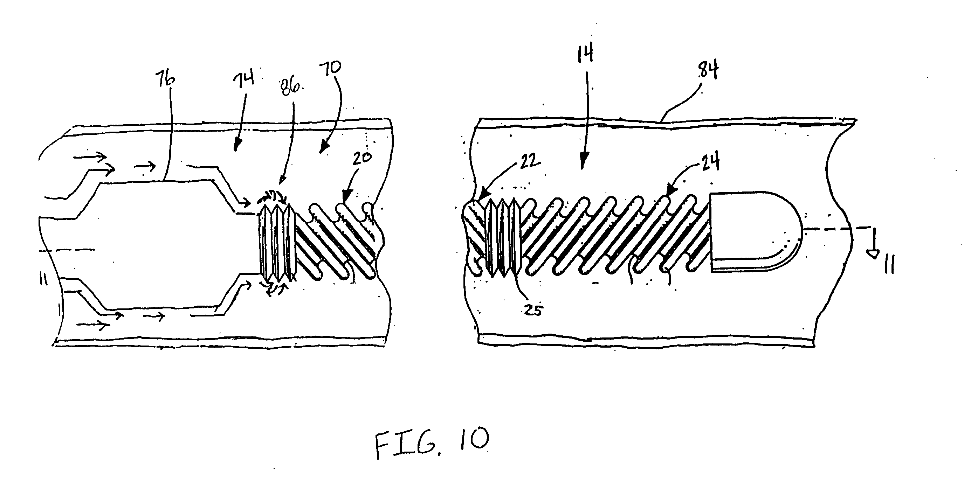

With reference to Figures 10-14, numerous embodiments of a heat transfer mechanism for selective heating or cooling of an organ will now be described. The heat transfer mechanism discussed with respect to Figures 10-14 is similar to the heat transfer element described with reference to Figures 4 and 8 above, but further includes a turbulence-enhancing element for enhancing the turbulent kinetic energy in the free stream and boundary layer of the heat transfer element 14. It will be shown that the turbulence-enhancing element may enhance turbulence around the heat transfer element 14 in numerous ways such as, but not by way of limitation, increasing the velocity of the blood contacting the heat transfer element 14, altering the normal direction of blood flow contacting the heat transfer element 14, and by increasing the level of turbulence in the blood flow before the blood reaches the heat transfer element 14, i.e., creating "pre-turbulence."

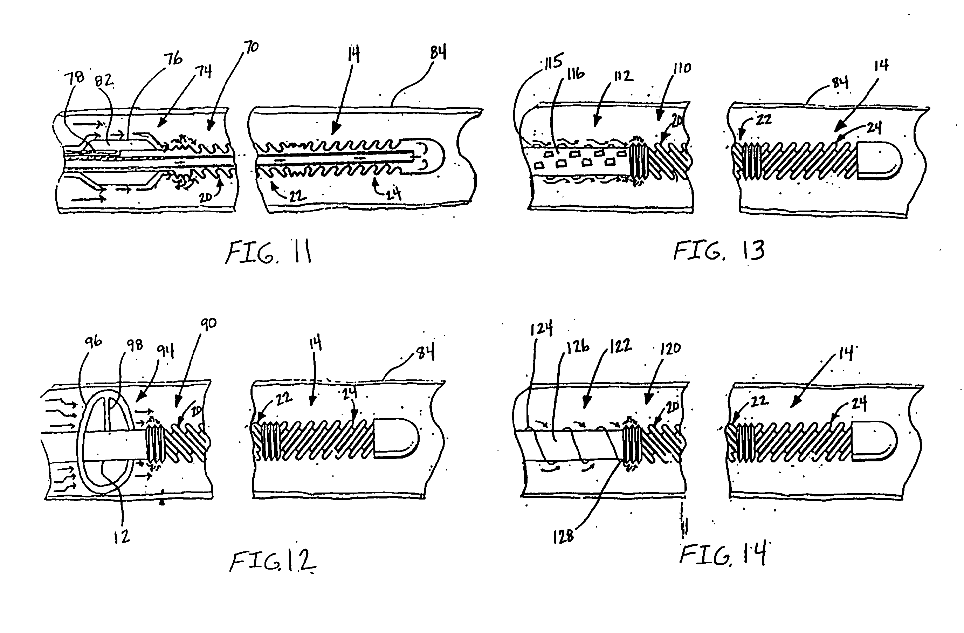

With reference to Figures 10 and 11, a heat transfer mechanism 70 constructed in accordance with an embodiment of the invention will now be described. The heat transfer mechanism 70 is located at a distal portion of a supply catheter 12. The heat transfer mechanism 70 includes a heat transfer element 14 located distally of a turbulence-enhancing element 74. The heat transfer element 14 may be the same as that described above with respect to Figures 4-7 and, thus, will not be described in any further detail. While for purposes of brevity the discussion herein is directed to a heat transfer element 14, it will be readily apparent to those skilled in the art that a heat transfer element other than that described with respect to Figures 4-7 may be used. For example, the heat transfer element 50 described with respect to Figures 8-9 may be used. In the embodiment shown in Figures 10 and 11, the turbulence-enhancing element 74 is an expandable micro-balloon 76. A lumen 78 (Figure 11) is located within the supply catheter 12 for expanding and contracting the micro-balloon 76 with a fluid, such as air. Although the micro-balloon 76 is described as being expanded and contracted using air, it will be readily apparent to those skilled in the art that other fluids, such as saline, may be used. The lumen 78 is in communication with a fluid source at a proximal end of the lumen 78 and in communication with an interior 82 of the micro-balloon 76 at a distal end. In an alternative embodiment of the invention, the catheter 12 may include more than one lumen for expanding the micro-balloon 76. A conventional control mechanism may be connected to the proximal end of the lumen 78 for controlling expansion and contraction of the micro-balloon 76. Examples of control mechanisms include, but not by way of limitation, a plunger, a squeezable bladder, or a pump.

The heat transfer mechanism 70 will now be generally described in use. The heat transfer mechanism 70 is positioned in a desired location in a patient's blood vessel 84, upstream from the desired organ to be cooled. During positioning of the heat transfer mechanism 70, the micro-balloon 76 is provided in a deflated or collapsed state to facilitate navigation of the catheter 12 and heat transfer mechanism 70 through the patient's vascular system. Once the heat transfer mechanism 70 is in position within the blood vessel, the micro-balloon 76 is expanded by filling it with fluid. In an expanded state, the micro-balloon 76 restricts the available blood flow volume in that region of the blood vessel and thus causes the blood adjacent the balloon 76 to travel at greater velocity compared to when the balloon 76 is in a collapsed state. However, as this blood passes the micro-balloon 76, the blood flow volume area is again expanded and the blood floods this volume where the heat transfer element 14 is located. Turbulence is enhanced along the heat transfer element, especially at a proximal portion 86 of the heat transfer element 14, by the changing direction of the velocity vectors of the blood flow contacting the segments 20. 22; 24. The blood contacts the successive alternating helical heat transfer segments 20, 22, 24, creating alternating flow that results in mixing the blood. This mixing action promotes high level turbulent kinetic energy to enhance the heat transfer rate between the heat transfer element 14 and the blood. The micro-balloon 76 further promotes or enhances this high level turbulent kinetic energy by increasing the velocity of the blood contacting the heat transfer element 14.

With reference to Fig. 12, a heat transfer mechanism 90 constructed in accordance with another embodiment of the invention will now be described. The heat transfer mechanism 90 includes a heat transfer element 14 similar to that described above and a turbulence-enhancing element 94 in the form of an expandable micro-ring balloon 96. An internal lumen similar to the lumen 78 described above with respect to Fig. 11 is located within the catheter 12 and in communication with a fluid source and control mechanism at a proximal end for controlling inflation and deflation of the micro-ring balloon 96. A feed lumen 98 extends radially from the catheter 12 and communicates a distal end of the internal lumen with the micro-ring balloon 96. The micro-ring balloon 96 functions in a similar manner to the micro-balloon 76 described above with respect to Figure 10, except the micro-ring balloon 96 cause blood to flow away from the blood vessel wall and into the heat transfer element 14, inducing additional turbulence. Although the micro-ring balloon 96 is shown in communication with a single lumen 98, it will be readily apparent to those skilled in the art that in an alternative embodiment, the balloon 96 may be in communication with multiple lumens. Multiple lumens provide the micro-ring balloon 96 with additional support and facilitate expansion and contraction of the micro-ring balloon 96 with the vessel 84.

With reference to Fig. 13, a heat transfer mechanism 110 constructed in accordance with a further embodiment of the invention will be described. The heat transfer mechanism 110 includes a heat transfer element 14 similar to that described above and a turbulence-enhancing element 112 in the form of axially staggered and circumferentially overlapping protrusions 115 located on an external surface 116 of the catheter 12. The protrusions 115 may be similar to the protrusions 54 described above with respect to Figures 8 and 9, except they are preferably located proximal of the heat transfer element 14 instead of on the heat transfer element 14. As the blood flows along the external surface 116, it collides with the staggered protrusions 115 and turbulent flow, i.e., "pre-turbulence" is initiated. As the blood divides and swirls around a staggered protrusion 115, it collides with another staggered protrusion 115 within its path, preventing the re-lamination of the flow and creating additional turbulence. The turbulent blood then contacts the successive alternating helical heat transfer segments 20-24, creating alternating flow that results in additional mixing of the blood. This mixing action promotes further high level turbulent kinetic energy to enhance the heat transfer rate between the heat transfer element 14 and the blood.

With reference to Fig. 14, a heat transfer mechanism 120 constructed in accordance with a further embodiment of the invention will be described. The heat transfer mechanism 120 includes a heat transfer element 14 similar to that described above and a turbulence-enhancing element 122 in the form of a temperature sensor wire 124 such as thermocouple wire helically wound around an external surface 126 of the catheter 12. The temperature sensor wire 124 may include a shrink wrap to hold the wire 124 in place. The temperature sensor wire 124 includes a temperature sensor 128 such as a thermocouple 128 in thermal contact with the heat transfer element 14. In an alternative embodiment, the thermocouple 128 may be replaced by a thermistor or similar temperature sensor coupled to a wire for measuring the temperature of the heat transfer element. The thermocouple 128 measures the temperature of the heat transfer element 14 for feedback control of the working fluid temperature. The thermocouple 128 may also be disposed distal of the heat transfer element 14 to measure the temperature of the blood downstream of the heat transfer element 14, e.g., measuring the temperature of the cooled blood. The helically wound thermocouple wire 124 causes blood to swirl over the external surface 126 of the catheter, re-directing the blood flow prior to contact with the heat transfer element 14. The re-direction and swirling of the blood flow further enhances the amount of turbulence created when the blood contacts the successive alternating helical heat transfer segments 20-24. In an alternative embodiment of the invention, a thick wire not used for measuring temperature may replace the thermocouple wire 124 proximal to the heat transfer element. In a further embodiment, a wire, e.g., thermocouple wire or thick wire, may be helically wrapped around a smooth exterior surface of a heat transfer element such as exterior surface 52 described above with respect to Figures 8 and 9. The helically wrapped wire would induce turbulent blood flow around the heat transfer element, enhancing heat transfer in this area.

In such wire-wrapped embodiments, it may be useful to have the pitch of the wire be such that turbulence is maintained without relamination of the flow. In other words, the wires should be wound in a close enough manner that turbulence is created and not merely "tripping."

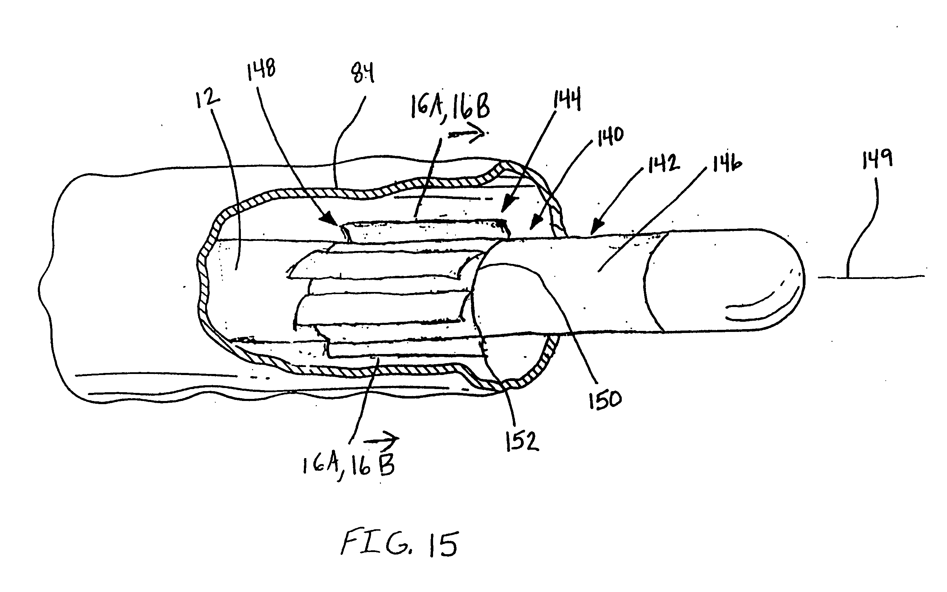

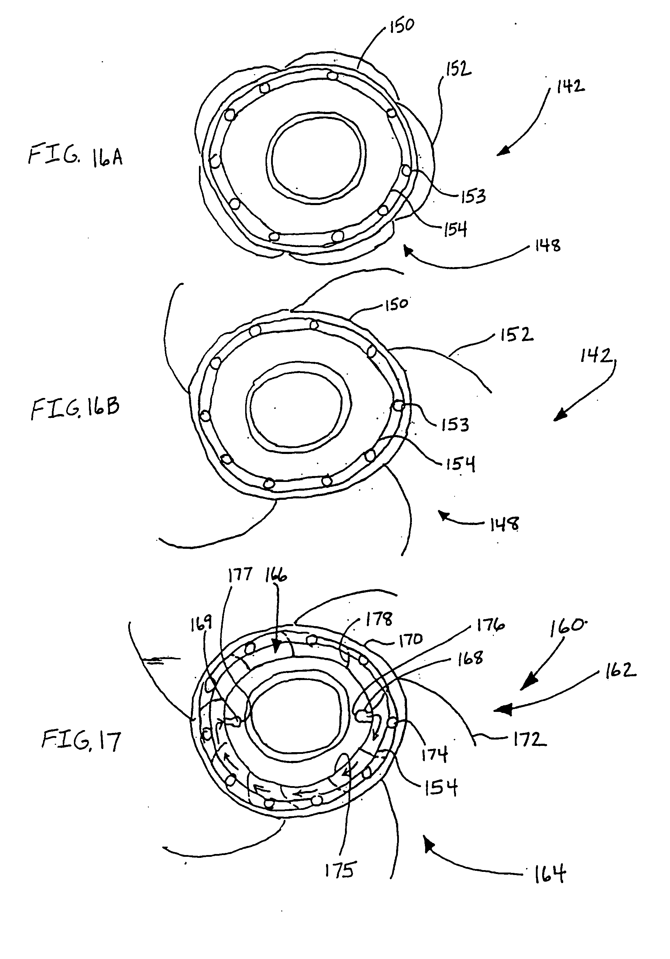

With reference to Figures 15, 16A and 16B, a heat transfer mechanism 140 constructed in accordance with an additional embodiment of the invention will be described. The heat transfer mechanism 140 includes a heat transfer element 142 and a turbulence-enhancing element 144. The heat transfer element 142 has a primarily smooth external surface 146 adapted to contact blood within the blood vessel 84. In an alternative embodiment, the heat transfer element 142 has turbulence-inducing features similar to those described above. The turbulence-enhancing element 144 includes a turbulence-generating fan 148. The fan 148 preferably rotates about an axis 149, coaxial with the axis of the catheter 12. The fan 148 includes a rotating hub 150 having multiple blades 152 extending therefrom. The fan 148 is adapted to spin upon influence of blood flow within the vessel 84. The blades 152 are constructed to move away from the hub 150 (FIG. 16B) upon influence of blood flow and towards the hub 150 (FIG. 16A), in a low profile configuration, when blood flow lessens or ceases. A low-profile configuration means that the blades 152 are located close enough to the external surface 146 of the catheter to prevent the fan 148 from catching upon and perhaps damaging the vasculature upon introduction and removal of the heat transfer element 142. Referring to Figures 16A and 16B, bearings 153 located between the hub 150 and an external portion 154 of the catheter 12 allow the fan 148 to rotate. The hub 150 is appropriately sealed with respect to the catheter 12 in order to prevent contamination of the blood and protect the bearings 152. The rotating fan 148 induces high level turbulent kinetic energy that enhances the heat transfer rate between the smooth heat transfer element 142 and the blood.

With reference to Figure 17, a heat transfer mechanism 160 constructed in accordance with an additional embodiment of the invention will be described. The heat transfer mechanism includes a heat transfer element 142 similar to that described above with respect to Figure 15 and a turbulence-enhancing element 162. The turbulence-enhancing element 162 includes a turbulence generating fan 164 similar to fan 148 described above, except the fan 164 includes an internal driving mechanism 166 for rotating the fan 164.

The driving mechanism 166 may include an input lumen 168 and an output lumen 169 in communication with a pump and fluid source at respective proximal ends of the lumens 168, 169 and a fluid drive tunnel 175 at respective distal ends 176, 177 of the lumens 168, 169. The fan 164 includes a rotating hub 170 with multiple blades 172 that move away from the hub 170 upon forced rotation of the fan 164 and towards the hub 170 when the fan 164 ceases rotation. Bearings 174 are located between the hub 170 and the external portion 154 of the catheter 12. The hub 170 is sealed with respect to the catheter 12 in order to prevent contamination of the blood and protect the bearings 174. The fan 164 includes internal blades 178 located within the fluid drive tunnel 175.

Pressurized fluid such as air is pumped through the input lumen 168 and into the fluid drive tunnel 175. Air flows through the fluid drive tunnel 175 in the direction of the arrows, causing the fan 164 to rotate via the internal blades 178. Air exits the fluid drive tunnel 175 through the output lumen 169.

The rotating fan 164 induces high level turbulent kinetic energy or mixing that enhances the heat transfer rate between the smooth heat transfer element 142 and the blood. It will be readily appreciated that multiple variations may exist on the heat transfer mechanism 160. For example, in an alternative embodiment, the internal driving mechanism 166 may be constructed so that the working fluid drives the fan 164 via the fluid drive tunnel 175 and internal blades 178. Other mechanical driving mechanisms may be used to drive the fan 164 such as, but not by way of limitation, a motor coupled to a rotatable drive shaft.

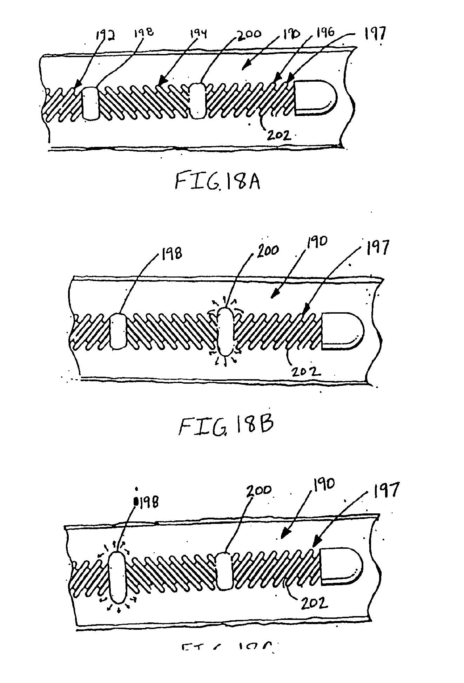

With reference to Figures 18A-18C, a heat transfer mechanism 190 constructed in accordance with an additional embodiment of the invention will be described. The heat transfer mechanism 190 includes a series of elongated, articulated heat transfer segments 192, 194, 196, which are similar to the segments 20, 22, 24 discussed above with respect to Figures 4-7, connected by flexible joints in the form of tubing sections 198, 200. The heat transfer segments 192, 194, 196 serve as a heat transfer element 197 for transferring heat between the blood flow and the working fluid. The tubing sections 198, 200 are made of a flexible biocompatible material such as a polymer that is seamless and nonporous. The tubing sections 198, 200 are adapted to bend, extend and compress, which increases the flexibility of the heat transfer element 197 so that it is more readily able to navigate through blood vessels. The tubing sections 198, 200 also provide axial compression of the heat transfer element 197, which can limit the trauma when the distal end of the heat transfer element 197 abuts a blood vessel wall. The tubing sections 198, 200 are also able to tolerate cryogenic temperatures without a loss of performance. During use of the heat transfer mechanism 190, working fluid is pulsed through an inner coaxial lumen of an inner tube and out of a distal end of the inner tube into an outer lumen (See, for example, Figure 5, inner coaxial lumen 40, inner tube 42, outer lumen 46). As the working fluid is pulsed through the outer lumen, heat is transferred from the working fluid to an exterior surface 202 of the heat transfer element 197. As the working fluid is pulsed through the inner lumen and outer lumen, the flexible tubing sections 198, 200, which include an internal area in fluid communication with the outer lumen, sequentially pulsate or expand, as shown in Figures 18B and 18C. The pulsating bellow sections 198, 200 transfer their vibrations to the blood flow, promoting turbulent kinetic energy as the blood flow contacts the heat transfer element 197. In addition, the expanded diameter of the flexible bellow sections 198, 200 caused by each pulsation promotes high level turbulent kinetic energy by increasing the velocity of the blood contacting the heat transfer element 197 in a manner similar to that described above for the micro-balloon 76.

In an alternative embodiment of the invention, the tubing sections 198, 200 may be replaced with micro-balloons, which are connected to separate lumens for individually controlling the pulsation of the balloon sections.

In an additional embodiment, a non-pulsing flow of working fluid may be employed. In this embodiment, the expanded flexible sections themselves cause turbulence in the blood flowing past the same.

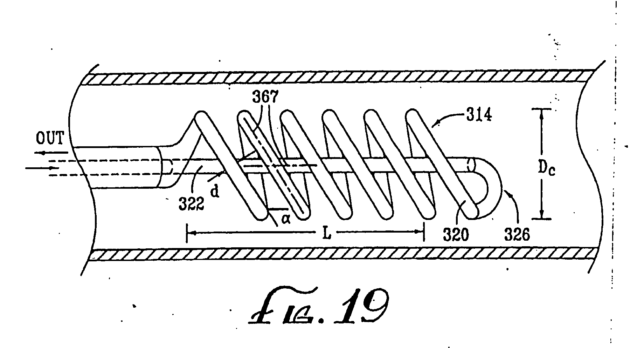

With reference generally to Figures 19-24, a heat transfer element according to an additional aspect of the present invention is made of a flexible material such as latex rubber. The latex rubber provides a high degree of flexibility which was previously achieved by articulation. The latex rubber further allows the heat transfer element to be made collapsible so that when deflated the same may be easily inserted into an artery. Insertion and location may be conveniently made by way of a guide catheter or guide wire. Following insertion and location in the desired artery, the heat transfer element may be inflated or expanded for use by a working fluid such as saline, water, perfluorocarbons, or other suitable fluids.

A heat transfer element made of a flexible material such as latex rubber generally has significantly less thermal conductivity than a heat transfer element made of metal. The present device compensates for this by enhancing the surface area available for heat transfer. This may be accomplished in two ways: by increasing the cross-sectional size and by increasing the length. Regarding the former, the device may be structured to be large when inflated, because when deflated the same may still be inserted into an artery. In fact, the device may be as large as the arterial wall, so long as a path for blood flow is allowed, because the flexibility of the device tends to prevent damage to the arterial wall even upon contact. Such paths are described below. Regarding the latter, the device may be configured to be long. One way to configure a long device is to taper the same so that the device may fit into distal arteries having reduced radii in a manner described below. The device further compensates for the reduced thermal conductivity by reducing the thickness of the heat transfer element wall.

Similar to the embodiments of the device described above, the embodiments discussed below use a heat transfer element design that produces a high level of turbulence in the free stream of the blood and in the working fluid. One embodiment of the invention forces a helical motion on the working fluid and imposes a helical barrier in the blood, causing turbulence similar to that described above with respect to Figures 4-7. In an alternative embodiment, the helical barrier is tapered. In another embodiment, a tapered inflatable heat transfer element has surface features to cause turbulence. As an example, the surface features may have a spiral shape. In all of these embodiments, the design forces a high level of turbulence in the free stream of the blood by causing the blood to navigate a tortuous path while passing through the artery. This tortuous path causes the blood to undergo violent accelerations resulting in turbulence.

In a further embodiment of the invention, a taper of an inflatable heat transfer element provides enough additional surface area per se to cause sufficient heat transfer. In all of the embodiments, the inflation is performed by the working fluid, such as water or saline.

With reference to Fig. 19, a side view is shown of an additional embodiment of a heat transfer element 314 according to an embodiment of the invention. The heat transfer element 314 is formed by an inlet lumen 322 and an outlet lumen 320. In this embodiment, the outlet lumen 320 is formed in a helix shape surrounding the inlet lumen 322, which is formed in a pipe shape. The names of the lumens are of course not limiting. It will be clear to one skilled in the art that the inlet lumen 322 may serve as an outlet and the outlet lumen 320 may serve as an inlet. It will also be clear that the heat transfer element is capable of both heating (by delivering heat to) and cooling (by removing heat from) a desired area.

The heat transfer element 314 is rigid but flexible so as to be insertable in an appropriate vessel by use of a guide catheter. Alternatively, the heat transfer element may employ a device for threading a guide wire therethrough to assist placement within an artery. The heat transfer element 314 has an inflated length of L, a helical diameter of D_{c}, a tubal diameter of d, and a helical angle of α. For example, D_{c} may be about 3.3 mm and d may be about 0.9 mm to 1 mm. Of course, the tubal diameter d need not be constant. For example, the diameter of the inlet lumen 322 may differ from that of the outlet lumen 314.

The shape of the outlet lumen 320 in Fig. 19 is helical. This helical shape presents a cylindrical obstacle, in cross-section, to the flow of blood. Such obstacles tend to create turbulence in the free stream of blood. In particular, the form of turbulence is believed to be the creation of von Karman vortices in the wake of the flow of blood, downstream of the cylindrical obstacles.

Typical inflatable materials are not highly thermally conductive. They are much less conductive than the metallic heat transfer elements described in the embodiments above. The difference in conductivity is compensated for in at least two ways in the present device. The material is made thinner and the heat transfer element is afforded a larger surface area. Regarding the former, the thickness may be less than about ½ mil for adequate cooling.

Thin inflatable materials, particularly those with large surface areas, may require a structure, such as a wire, within their interiors to maintain their approximate uninflated positions so that upon inflation, the proper form is achieved. Thus, a wire structure 367 is shown in Fig. 19 which may be advantageously disposed within the inflatable material to perform such a function.

Another consideration is the angle α of the helix. Angle α should be determined to optimize the helical motion of the blood around the lumens 320 and 322, enhancing heat transfer. Of course, angle α should also be determined to optimize the helical motion of the working fluid within the lumens 320 and 322. The helical motion of the working fluid within the lumens 320 and 322 increases the turbulence in the working fluid by creating secondary motions. In particular, helical motion of a fluid in a pipe induces two counter-rotating secondary flows.

An enhancement of h̅_{c} would be obtained in this system, and this enhancement may be described by a Nusselt number Nu of up to about 10 or even more.

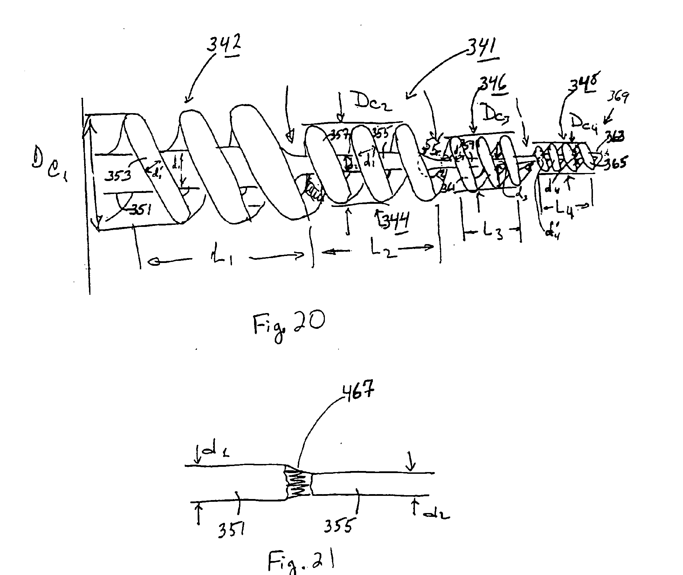

An alternative embodiment of the device, shown in a side view in Fig. 20, illustrates a heat transfer element 341 with a surface area enhancement. Increasing the surface area of the inflatable material enhances heat transfer. The heat transfer element 314 includes a series of coils or helices of different coil diameters and tubal diameters. It is not strictly necessary that the tubal diameters differ, but it is likely that commercially realizable systems will have differing tubal diameters. The heat transfer element 341 may taper either continuously or segmentally.

This alternative embodiment enhances surface area in two ways. First, the use of smaller diameter lumens enhances the overall surface-to-volume ratio. Second, the use of progressively smaller (i.e., tapered) lumens allows a distal end 369 to be inserted further into an artery than would be possible with the embodiment of Fig. 19.

In the embodiment of Fig. 20, a first coil segment 342 is shown having length L, and diameter D_{C1}. The first coil segment 342 is formed of an inlet lumen 351 having diameter d₁ and an outlet lumen 353 having diameter d₁'. In the first coil segment, as well as the others, the outlet lumen need not immediately drain the inlet lumen. In Fig. 20, the inlet lumen for each segment feeds the inlet lumen of the succeeding segment except for an inlet lumen adjacent a distal end 369 of the heat transfer element 341 which directly feeds its corresponding outlet lumen.

A separate embodiment may also be constructed in which the inlet lumens each provide working fluid to their corresponding outlet lumens. In this embodiment, either a separate lumen needs to be provided to drain each outlet lumen or each outlet lumen drains into the adjacent outlet lumen. This embodiment has the advantage that an opposite helicity may be accorded each successive segment. The opposite helicities in turn enhance the turbulence of the working fluid flowing past them.

A second coil segment 344 is shown having length L₂ and diameter D_{C2}. The second coil segment 344 is formed of an inlet lumen 355 having diameter d₂ and an outlet lumen 357 having diameter d₂'. A third coil segment 346 is shown having length L₃ and diameter D_{C3}.

The third coil segment 346 is formed of an inlet lumen 359 having diameter d₃ and an outlet lumen 361 having diameter d₃'.

Likewise, a fourth coil segment 348 is shown having length L, and diameter D_{C4}. The fourth coil segment 348 is formed of an inlet lumen 363 having diameter d₄ and an outlet lumen 365 having diameter d₄'. The diameters of the lumens, especially that of the lumen located at or near distal end 369, should be large enough to not restrict the flow of the working fluid within them. Of course, any number of lumens may be provided depending on the requirements of the user.

Fig. 21 shows the connection between two adjacent inlet lumens 351 and 355. A joint 467 is shown coupling the two lumens. The construction of the joint may be by way of variations in stress, hardening, etc.

An advantage to this alternative embodiment arises from the smaller diameters of the distal segments. The heat transfer element of Fig. 20 may be placed in smaller workspaces than the heat transfer element of Fig. 19. For example, a treatment for brain trauma may include placement of a cooling device in the internal carotid artery of a patient. As noted above, the common carotid artery feeds the internal carotid artery. In some patients, the heat transfer element of Fig. 19 may not fit in the internal carotid artery. Similarly, the first coil segment of the heat transfer element in Fig. 20 may not easily fit in the internal carotid artery, although the second, third, and fourth segments may fit. Thus, in the embodiment of Fig. 20, the first coil segment may remain in the common carotid artery while the segments of smaller diameter (the second, third, and fourth) may be placed in the internal carotid artery. In fact, in this embodiment, D_{C1} may be large, such as 5-6 mm. The overall length of the heat transfer element 341 may be, e.g., about 20 to 25 cm.

An additional advantage was mentioned above. The surface area of the alternative embodiment of Fig. 20 may be substantially larger than that of the embodiment of Fig. 19, resulting in significantly enhanced heat transfer. For example, the enhancement in surface area may be substantial, such as up to or even more than three times compared to the surface area of the device of the application incorporated by reference above. An additional advantage of both embodiments is that the helical rounded shape allows atraumatic insertion into cylindrical cavities such as, e.g., arteries.

The embodiment of Fig. 20 may result in an Nu from 1 up to about 50.

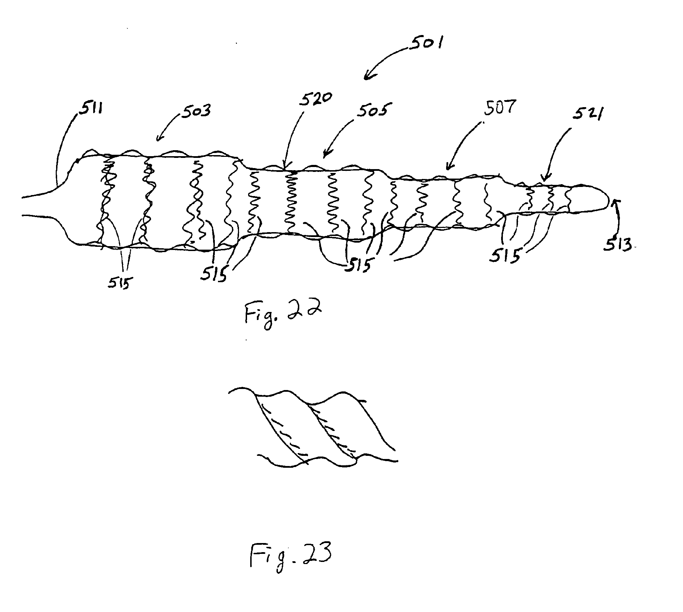

Fig. 22 shows a further embodiment of the device employing surface features rather than overall shape to induce turbulence. In particular, Fig. 22 shows a heat transfer element 501 having an inlet lumen (not shown) and an outlet inflatable lumen 520 having four segments 503, 505, 507, and 521. Segment 503 is adjacent a proximal end 511 and segment 521 is adjacent a distal end 513. The segments are arranged having reducing radii in the direction of the proximal end to the distal end. In a manner similar to that of the embodiment of Fig. 20, the feature of reducing radii allows insertion of the heat transfer element into small work places such as small arteries.

Heat transfer element 501 has a number of surface features 515 disposed thereon. The surface features 515 may be constructed with, e.g., various hardening treatments applied to the heat transfer element 501, or alternatively by injection molding. The hardening treatments may result in a wavy or corrugated surface to the exterior of heat transfer element 501. The hardening treatments may further result in a wavy or corrugated surface to the interior of heat transfer element 501. Fig. 23 shows a variation of this embodiment, in which a fabrication process is used which results in a spiral or helical shape to the surface features.

The embodiment of Fig. 22 may result in an Nu of about 1 to 50.

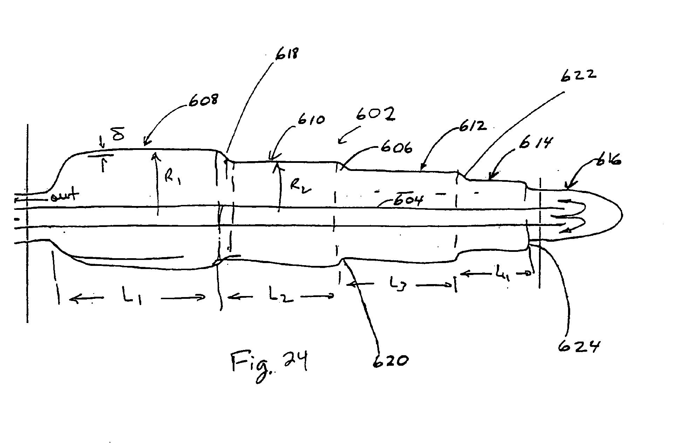

In some situations, an enhanced surface area alone, without the creation of additional turbulence, may result in sufficient heat transfer to cool the blood. With reference to Fig. 24, a heat transfer element 602 is shown having an inlet lumen 604 and an outlet lumen 606. The inlet lumen 604 provides a working fluid to the heat transfer element 602 and outlet lumen 606 drains the working fluid from the same. The functions may, of course, be reversed. The heat transfer element 602 is further divided into five segments, although more or less may be provided as dictated by requirements of the user. The five segments in Fig. 24 are denoted segments 608, 610, 612, 614, and 616. In Fig. 24, the segment 608 has a first and largest radius R₁, followed by corresponding radii for segments 610, 612, 614, and 616. Segment 616 has a second and smallest radius. The length of the segment 608 is L₁, followed by corresponding lengths for segments 610, 612, 614, and 616.

A purely tapered (nonsegmented) form may replace the tapered segmental form, but the former may be more difficult to manufacture. In either case, the tapered form allows the heat transfer element 602 to be disposed in small arteries, i.e., arteries with radii smaller than R₁. A sufficient surface area is thus afforded even in very small arteries to provide the required heat transfer.

The surface area and thus the size of the device should be substantial to provide the necessary heat transfer. Example dimensions for a three-segmented tapered form may be as follows: L₁ = 10 cm, R₁ = 2.5 mm; L₂ = 10 cm, R₂ = 1.65 mm, L₃ = 5 cm, R₃ = 1 mm. Such a heat transfer element would have an overall length of 25 cm and a surface area of 3 x 10⁻⁴ m².

The embodiment of Fig. 24 results in an enhancement of the heat transfer rate of up to about 300% due to the increased surface area S alone.

A variation of the embodiment of Fig. 24 includes placing at least one turbulence-inducing surface feature within the interior of the outlet lumen 606. This surface feature may induce turbulence in the working fluid, thereby increasing the convective heat transfer rate in the manner described above.

Another variation of the embodiment of Fig. 24 involves reducing the joint diameter between segments (not shown). For example, the inflatable material may be formed such that joints 618, 620, 622, and 624 have a diameter only slightly greater than that of the inlet lumen 604. In other words, the heat transfer element 602 has a tapered "sausage" shape.

In all of the embodiments, the inflatable material may be formed from seamless and nonporous materials which are therefore impermeable to gas. Impermeability can be particularly important depending on the type of working fluid which is cycled through the heat transfer element. For example, the inflatable or expandable material may be latex or other such rubber materials, or alternatively of any other material with similar properties under inflation. The flexible material allows the heat transfer element to bend, extend and compress so that it is more readily able to navigate through tiny blood vessels. The material also provides for axial compression of the heat transfer element which can limit the trauma when the distal end of the heat transfer element 314 abuts a blood vessel wall. The material should be chosen to tolerate temperatures in the range of-1°C to 37°C, or even higher in the case of blood heating, without a loss of performance.

As discussed above, it may be desirable to treat the surface of the heat transfer element to avoid clot formation because the heat transfer element may dwell within the blood vessel for extended periods of time, such as 24-48 hours or even longer. One means by which to prevent thrombus formation is to bind an antithrombogenic agent such as heparin to the surface of the heat transfer element.

With reference back to Fig. 19, a method in accordance with a further aspect of the invention will be described. A description with reference to the embodiment of Fig. 20 is analogous. A guide catheter or wire may be disposed up to or near the area to be cooled or heated. The case of a guide catheter will be discussed here. The heat transfer element may be fed through the guide catheter to the area. Alternatively, the heat transfer element may form a portion of the guide catheter or vice versa. For example, a portion of the interior of the guide catheter may form the return lumen for the working fluid. In any case, the movement of the heat transfer element is made significantly more convenient by the flexibility of the heat transfer element as has been described above.

Once the heat transfer element 314 is in place, a working fluid such as saline or other aqueous solution may be circulated through the heat transfer element 314 to inflate the same. Fluid flows from a supply catheter into the inlet lumen 322. At the distal end 326 of the heat transfer element 314, the working fluid exits the inlet lumen 322 and enters the outlet lumen 320.

In the case of the embodiment of Fig. 22. for which the description of Figure 8 is analogous, the working fluid exits the inlet lumen and enters an outlet inflatable or expandable lumen 520 having segments 503, 505, 507, and 521. As the working fluid flows through the outlet lumen 520, heat is transferred from the exterior surface of the heat transfer element 501 to the working fluid. The temperature of the external surface may reach very close to the temperature of the working fluid because the heat transfer element 501 is constructed from very thin material.

The working fluids that may be employed in the device include water, saline or other fluids which remain liquid at the temperatures used. Other coolants, such as freon, undergo nucleated boiling and may create turbulence through a different mechanism. Saline is a safe coolant because it is non-toxic and leakage of saline does not result in a gas embolism which may occur with the use of boiling refrigerants.

As discussed above, by enhancing turbulence in the coolant, the coolant can be delivered to the heat transfer element at a warmer temperature and still achieve the necessary heat transfer rate. In particular, the enhanced heat transfer characteristics of the internal structure allow the working fluid to be delivered to the heat transfer element at lower flow rates and lower pressures. This is advantageous because high pressures may stiffen the heat transfer element and cause the same to push against the wall of the vessel, thereby shielding part of the heat transfer unit from the blood. Such pressures are unlikely to damage the walls of the vessel because of the increased flexibility of the inflated device. The increased heat transfer characteristics allow the pressure of the working fluid to be delivered at pressures as low as 5 atmospheres, 3 atmospheres, 2 atmospheres or even less than 1 atmosphere.

In a preferred embodiment, the heat transfer element creates a turbulence intensity greater than 0.05 in order to create the desired level of turbulence in the entire blood stream during the whole cardiac cycle. The turbulence intensity may be greater than 0.055, 0.06, 0.07 or up to 0.10 or 0.20 or even greater.

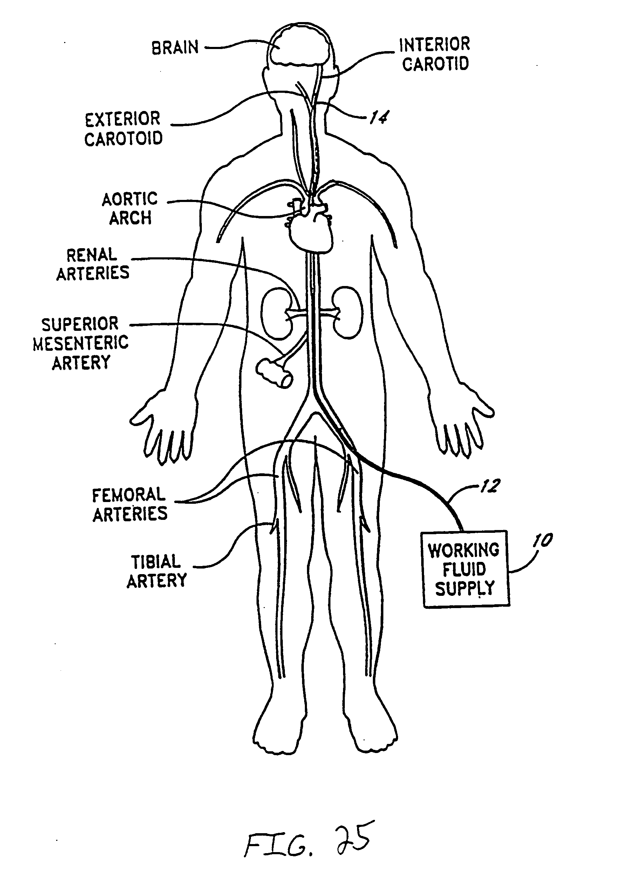

Figure 25 is a schematic representation of the invention being used to cool the brain of a patient. The selective organ hypothermia apparatus shown in Figure 25 includes a working fluid supply 10, preferably supplying a chilled liquid such as water alcohol or a halogenated hydrocarbon, a supply catheter 12 and the heat transfer element 14. Although the heat transfer element is identified with the reference numeral 14, it will be readily understood by those skilled in the art that the method described below may apply to any of the heat transfer elements described above as well as heat transfer elements not described herein. The working fluid supply 10 preferably supplies fluid via a pump (not shown). The supply catheter 12 has a coaxial construction. An inner coaxial lumen within the supply catheter 12 receives coolant from the working fluid supply 10. The coolant travels the length of the supply catheter 12 to the heat transfer element 14 which serves as the cooling tip of the catheter. At the distal end of the heat transfer element 14, the coolant exits the interior lumen and traverses the length of the heat transfer element 14 in order to decrease the temperature of the heat transfer element 14. The coolant then traverses an outer lumen of the supply catheter 12 so that it may be disposed of or recirculated. The supply catheter 12 is a flexible catheter having a diameter sufficiently small to allow its distal end to be inserted percutaneously into an accessible artery such as the femoral artery of a patient as shown in Figure 25. The supply catheter 12 is sufficiently long to allow the heat transfer element 14 at the distal end of the supply catheter 12 to be passed through the vascular system of the patient and placed in the internal carotid artery or other small artery. The method of inserting the catheter into the patient and routing the heat transfer element 14 into a selected artery is well known in the art.

Although the working fluid supply 10 is shown as an exemplary cooling device, other devices and working fluids may be used. For example, in order to provide cooling, freon, perflourocarbon or saline may be used.

The heat transfer element of the present invention can absorb or provide over 75 Watts of heat to the blood stream and may absorb or provide as much as 100 Watts, 150 Watts, 170 Watts or more. For example, a heat transfer element with a diameter of 4 mm and a length of approximately 10 cm using ordinary saline solution chilled so that the surface temperature of the heat transfer element is approximately 5° C and pressurized at 2 atmospheres can absorb about 100 Watts of energy from the bloodstream. Smaller geometry heat transfer elements may be developed for use with smaller organs which provide 60 Watts, 50 Watts, 25 Watts or less of heat transfer.

The practice of the present invention is illustrated in the following non-limiting example.

### Exemplary Procedure

1. The patient is initially assessed, resuscitated, and stabilized.
2. The procedure is carried out in an angiography suite or surgical suite equipped with fluoroscopy.
3. Because the catheter is placed into the common carotid artery, it is important to determine the presence of stenotic atheromatous lesions. A carotid duplex (doppler/ultrasound) scan can quickly and non-invasively make this determinations. The ideal location for placement of the catheter is in the left carotid so this may be scanned first. If disease is present, then the right carotid artery can be assessed. This test can be used to detect the presence of proximal common carotid lesions by observing the slope of the systolic upstroke and the shape of the pulsation. Although these lesions are rare, they could inhibit the placement of the catheter. Examination of the peak blood flow velocities in the internal carotid can determine the presence of internal carotid artery lesions. Although the catheter is placed proximally to such lesions, the catheter may exacerbate the compromised blood flow created by these lesions. Peak systolic velocities greater that 130 cm/sec and peak diastolic velocities> 100 cm/sec in the internal indicate the presence of at least 70% stenosis. Stenosis of 70% or more may warrant the placement of a stent to open up the internal artery diameter.
4. The ultrasound can also be used to determine the vessel diameter and the blood flow and the catheter with the appropriately sized heat transfer element could be selected.
5. After assessment of the arteries, the patients inguinal region is sterilely prepped and infiltrated with lidocaine.
6. The femoral artery is cannulated and a guide wire may be inserted to the desired carotid artery. Placement of the guide wire is confirmed with fluoroscopy.
7. An angiographic catheter can be fed over the wire and contrast media injected into the artery to further to assess the anatomy of the carotid.
8. Alternatively, the femoral artery is cannulated and a 10-12.5 french (f) introducer sheath is placed.
9. A guide catheter is placed into the desired common carotid artery. If a guiding catheter is placed, it can be used to deliver contrast media directly to further assess carotid anatomy.
10. A 10 f -12 f (3.3- 4.0 mm) (approximate) cooling catheter is subsequently filled with saline and all air bubbles are removed. In the case of inflatable catheter embodiments of the invention described above, the saline filling step may be performed after placement of the catheter in the appropriate artery.
11. The cooling catheter is placed into the carotid artery via the guiding catheter or over the guidewire. Placement is confirmed with fluoroscopy.
12. Alternatively, the cooling catheter tip is shaped (angled or curved approximately 45 degrees), and the cooling catheter shaft has sufficient pushability and torqueability to be placed in the carotid without the aid of a guide wire or guide catheter.
13. The cooling catheter is connected to a pump circuit also filled with saline and free from air bubbles. The pump circuit has a heat exchange section that is immersed into a water bath and tubing that is connected to a peristaltic pump. The water bath is chilled to approximately 0° C.
14. Cooling is initiated by starting the pump mechanism. The saline within the cooling catheter is circulated at 5 cc/sec. The saline travels through the heat exchanger in the chilled water bath and is cooled to approximately 1° C.
15. The working fluid subsequently enters the cooling catheter where it is delivered to the heat transfer element. The saline is warmed to approximately 5-7° C as it travels along the inner lumen of the catheter shaft to the end of the heat transfer element.
16. The saline then flows back through the heat transfer element in contact with the inner metallic surface. The saline is further warmed in the heat transfer element to 12-15° C, and in the process, heat is absorbed from the blood, cooling the blood to 30° C to 32° C.
17. The chilled blood then goes on to chill the brain. It is estimated that 15-30 minutes will be required to cool the brain to 30 to 32° C.
18. The warmed saline travels back down the outer lumen of the catheter shaft and back to the chilled water bath where it is cooled to 1° C.
19. The pressure drops along the length of the circuit are estimated to be 2-3 atmospheres.
20. The cooling can be adjusted by increasing or decreasing the flow rate of the saline. Monitoring of the temperature drop of the saline along the heat transfer element will allow the flow to be adjusted to maintain the desired cooling effect.
21. The catheter is left in place to provide cooling for 12 to 24 hours.
22. If desired, warm saline can be circulated to promote warming of the brain at the end of the therapeutic cooling period.

While the particular invention as herein shown and disclosed in detail is fully capable of obtaining the objects and providing the advantages stated, it is to be understood that this disclosure is merely illustrative of the presently preferred embodiments of the invention and that no limitations are intended other than as described in the appended claims.

## Claims

1. A selective organ heat transfer device, including:
a catheter (12) capable of insertion into a selected blood vessel in the vascular system of a patient and
a heat transfer element (14) attached to a distal portion of the catheter (12), the heat transfer device including a mixing-enhancing element (74) attached to a distal portion of the catheter (12) and adapted to enhance mixed blood flow along said heat transfer element (14),
wherein said mixing-enhancing element (74) includes a plurality of exterior surface irregularities (26, 28, 30', 32, 34, 36) formed on a plurality of heat transfer segments (20, 22, 24), **characterised in that** said surface irregularities (26, 28, 30, 32, 34, 36) are shaped and arranged to create repetitively changing directions of flow in surrounding fluid said surface irregularities having a depth at least equal to the boundary layer thickness of flow between said heat transfer element and the feeding artery.

2. A heat transfer device as recited in claim 1, wherein said surface irregularities (26; 28, 30, 32, 34, 36) comprise a helical ridge (28, 32, 36) and a helical groove (26, 30, 34) formed on each said heat transfer segment (20, 22, 24), and said helical ridge (28, 32, 36) on each said heat transfer segment (20, 22, 24) has an opposite helical twist to said helical ridges (28, 32, 36) on adjacent said heat transfer segments (20, 22, 24).

3. A heat transfer device as recited in claim 1, wherein said mixing-enhancing element (74) is located proximal of said heat transfer element (14).

4. A heat transfer device as recited in claim 3, wherein said mixing-enhancing element (74) includes an expandable micro-balloon (16).

5. A heat transfer device as recited in claim 3, wherein said mixing-enhancing element (74) includes an expandable micro-ring balloon (16).

6. A heat transfer device as recited in claim 3, wherein said mixing-enhancing element (74) includes multiple axially staggered and circumferentially overlapping protrusions (54) located along said exterior surface.

7. A heat transfer device as recited in claim 3, wherein said mixing-enhancing element (74) includes a temperature sensor wire (124) helically wound around said exterior surface and having a temperature sensor (128) in thermal contact with said heat transfer element (14) for feedback control of said heat transfer element (14).

8. A heat transfer device as recited in claim 7, wherein said temperature sensor (128) is a member from the group consisting of thermocouple and thermistor.

9. A heat transfer device as recited in claim 3, wherein said catheter (12) includes a longitudinal axis and said mixing-enhancing element (74) includes a fan (164) adapted to rotate about an axis coaxial with said longitudinal axis.of said catheter.

10. A heat transfer device as recited in claim 9, further including a driving mechanism (166) adapted to rotate said fan (164).

11. A heat transfer device according to any of the preceding claims, wherein the catheter (20) comprises :
an inlet lumen (322; 359; 363; 604); and
an outlet lumen (314; 320; 361; 365; 520; 606) the outlet lumen coupled to the inlet lumen so as to transfer a working fluid between the two, one of the inlet lumen or outlet lumen having a helical shape,
wherein the inlet lumen and the outlet lumen are made of a flexible material, the flexible material being a material capable of undergoing inflation.

12. A heat transfer device of claim 11, further comprising more than one outlet lumen.

13. A heat transfer device of claim 12, wherein each outlet lumen helically encircles the inlet lumen.

14. A heat transfer device of any one of claims 11 to 13, wherein the working fluid is saline.

15. A heat transfer device of any one of claims 11 to 14, further comprising a wire (124) at least partially disposed within the inlet lumen or the outlet lumen.

16. A heat transfer device of any one of claims 11 to 15, wherein a length of the inlet lumen is between about 5 and 30 centimeters.

17. A heat transfer device of any one of claims 11 to 16, further comprising a working fluid supply including a pump, and wherein the pump circulates the working fluid.

18. A heat transfer device of claim 17, wherein the working fluid supply is configured to produce a pressurized working fluid at a temperature of between about -3°C and 36°C and at a pressure below about 5 atmospheres.

19. A heat transfer device of any one of claims 11 to 18, wherein the outlet lumen helically encircles the inlet lumen.

20. A heat transfer device of claim 19, wherein the outlet lumen includes a surface coating or treatment to inhibit clot formation.

## Patentansprüche

1. Wärmeübertragungsvorrichtung für ein ausgewähltes Organ, die Folgendes umfasst:
einen Katheter (12), das in ein ausgewähltes Blutgefäß im Gefäßsystem eines Patienten eingeführt werden kann; und
ein Wärmeübertragungselement (14), das an einem distalen Teil des Katheters (12) befestigt ist, wobei die Wärmeübertragungsvorrichtung Folgendes umfasst:
ein mischungsförderndes Element (74), das an dem distalen Teil des Katheters (12) befestigt und in der Lage ist, das Fließen gemischten Blutes längs dem genannten Wärmeübertragungselement (14) zu fördern, wobei das genannte mischungsfördernde Element (74) eine Vielzahl von Unregelmäßigkeiten der Außenfläche (26, 28, 30, 32, 34, 36) aufweist, die auf einer Vielzahl von Wärmeübertragungssegmenten (20, 22, 24) ausgebildet sind,
**dadurch gekennzeichnet, dass** die genannten Unregelmäßigkeiten der Oberfläche (26, 28, 30, 32, 34, 36) derart geformt und angeordnet sind, dass sie wiederholt wechselnde Strömungsrichtungen in der umgebenden Flüssigkeit bewirken, wobei die genannten Unregelmäßigkeiten der Oberfläche eine Tiefe aufweisen, die mindestens der Grenzschichtdicke der Strömung zwischen dem genannten Wärmeübertragungselement und der zuführenden Arterie entspricht.

2. Wärmeübertragungsvorrichtung nach Anspruch 1, wobei die genannten Unregelmäßigkeiten der Oberfläche (26, 28, 30, 32, 34, 36) eine spiralförmige Erhebung (28, 32, 36) und eine spiralförmige Vertiefung (26, 30, 34) umfassen, die auf jedem der genannten Wärmeübertragungssegmente (20, 22, 24) ausgebildet sind, und wobei die genannte spiralförmige Erhebung (28, 32, 36) auf jedem der genannten Wärmeübertragungssegmente (20, 22, 24) eine den genannten spiralförmigen Erhebungen (28, 32, 36) auf den benachbarten genannten Wärmeübertragungssegmenten (20, 22, 24) entgegengesetzte spiralförmige Windung aufweist.

3. Wärmeübertragungsvorrichtung nach Anspruch 1, wobei sich das genannte mischungsfördernde Element (74) proximal dem genannten Wärmeübertragungselement (14) befindet.

4. Wärmeübertragungsvorrichtung nach Anspruch 3, wobei das genannte mischungsfördernde Element (74) einen expandierbaren Mikroballon (16) umfasst.

5. Wärmeübertragungsvorrichtung nach Anspruch 3, wobei das genannte mischungsfördernde Element (74) einen expandierbaren Mikroringballon (16) umfasst.

6. Wärmeübertragungsvorrichtung nach Anspruch 3, wobei das genannte mischungsfördernde Element (74) mehrere axial gestaffelte und in Umfangsrichtung überlappende Vorsprünge (54) umfasst, die längs der genannten Außenfläche angeordnet sind.

7. Wärmeübertragungsvorrichtung nach Anspruch 3, wobei das genannte mischungsfördernde Element (74) einen Temperaturfühlerdraht (124) umfasst, der spiralförmig um die genannte Außenfläche gewickelt und mit einem Temperaturfühler (128) versehen ist, der sich im thermischen Kontakt mit dem genannten Wärmeübertragungselement (14) befindet, zur Regelung des genannten Wärmeübertragungselements (14).

8. Wärmeübertragungsvorrichtung nach Anspruch 7, wobei der genannte Temperaturfühler (128) ein Teil der Gruppe bestehend aus Thermoelement und Thermistor ist.

9. Wärmeübertragungsvorrichtung nach Anspruch 3, wobei das genannte Katheter (12) eine Längsachse umfasst und das genannte mischungsfördernde Element (74) einen Lüfter (164) umfasst, der so ausgelegt ist, dass er sich um eine Achse dreht, die koaxial zu der genannten Längsachse des genannten Katheters verläuft.

10. Wärmeübertragungsvorrichtung nach Anspruch 9, die ferner einen Antriebsmechanismus (166) umfasst, der so ausgelegt ist, dass er den genannten Lüfter (164) dreht.

11. Wärmeübertragungsvorrichtung nach einem der vorhergehenden Ansprüche, wobei der Katheter (20) Folgendes umfasst:
ein Einlasslumen (322, 359, 363, 604); und
ein Auslasslumen (314, 320, 361, 365, 520, 606), wobei das Auslasslumen mit dem Einlasslumen derart gekoppelt ist, dass eine Arbeitsflüssigkeit zwischen den beiden übertragen wird, wobei entweder das Einlasslumen oder Auslasslumen eine Spiralform aufweist, wobei das Einlasslumen und das Auslasslumen aus flexiblem Material bestehen, das ein Material ist, das einer Inflation unterzogen werden kann.

12. Wärmeübertragungsvorrichtung nach Anspruch 11, die ferner mehr als ein Auslasslumen umfasst.

13. Wärmeübertragungsvorrichtung nach Anspruch 12, wobei jedes Auslasslumen das Einlasslumen spiralförmig umgibt.

14. Wärmeübertragungsvorrichtung nach einem der Ansprüche 11 bis 13, wobei die Arbeitsflüssigkeit salzhaltig ist.

15. Wärmeübertragungsvorrichtung nach einem der Ansprüche 11 bis 14, die ferner einen Draht (124) umfasst, der zumindest teilweise innerhalb des Einlasslumens oder des Auslasslumens angeordnet ist.

16. Wärmeübertragungsvorrichtung nach einem der Ansprüche 11 bis 15, wobei die Länge des Einlasslumens zwischen ungefähr 5 und 30 cm liegt.

17. Wärmeübertragungsvorrichtung nach einem der Ansprüche 11 bis 16, die ferner eine Arbeitsflüssigkeitsversorgung mit einer Pumpe umfasst, wobei die Pumpe die Arbeitsflüssigkeit zirkuliert.

18. Wärmeübertragungsvorrichtung nach Anspruch 17, wobei die Arbeitsflüssigkeitsversorgung so konfiguriert ist, dass sie eine mit Druck beaufschlagte Arbeitsflüssigkeit mit einer Temperatur zwischen ungefähr -3°C und 36°C und einem Druck unter ungefähr 5 Atmosphären erzeugt.

19. Wärmeübertragungsvorrichtung nach einem der Ansprüche 11 bis 18, wobei das Auslasslumen das Einlasslumen spiralförmig umgibt.

20. Wärmeübertragungsvorrichtung nach Anspruch 19, wobei das Auslasslumen eine Oberflächenbeschichtung oder -behandlung aufweist, die Klumpenbildung verhindert.

## Revendications

1. Dispositif de transfert de chaleur d'organe sélectif, comprenant :
un cathéter (12) capable d'être inséré dans un vaisseau sanguin sélectionné dans le système vasculaire d'un patient; et
un élément de transfert de chaleur (14) attaché à une portion distale du cathéter (12), le dispositif de transfert de chaleur comprenant :
un élément d'amélioration de mélange (74) attaché à une portion distale du cathéter (12) et apte à améliorer l'écoulement sanguin mélangé le long dudit élément de transfert de chaleur (14), dans lequel ledit élément d'amélioration de mélange (14) comprend une pluralité d'irrégularités de surface extérieure (26, 28, 30, 32, 34, 36) formées sur une pluralité de segments de transfert de chaleur (20, 22, 24),
**caractérisé en ce que** lesdites irrégularités de surface (26, 28, 30, 32, 34, 36) sont formées et agencées pour créer des directions changeant à répétition d'écoulement de fluide environnant, lesdites irrégularités de surface ayant une profondeur au moins égale à l'épaisseur de couche de frontière d'écoulement entre ledit élément de transfert de chaleur et l'artère d'alimentation.

2. Dispositif de transfert de chaleur selon la revendication 1, dans lequel lesdites irrégularités de surface (26, 28, 30, 32, 34, 36) comprennent une crête hélicoïdale (28, 32, 36) et une rainure hélicoïdale (26, 30, 34) formées sur chacun desdits segments de transfert de chaleur (20, 22, 24), et ladite rainure hélicoïdale (28, 32, 36) sur chacun desdits segments de transfert de chaleur (20, 22, 24) a une torsion hélicoïdale opposée aux dites crêtes hélicoïdales (28, 32, 36) sur lesdits segments de transfert de chaleur adjacents (20, 22, 24).

3. Dispositif de transfert de chaleur selon la revendication 1, dans lequel ledit élément d'amélioration de mélange (74) est situé à proximité dudit élément de transfert de chaleur (14).

4. Dispositif de transfert de chaleur selon la revendication 3, dans lequel ledit élément d'amélioration de mélange (74) comprend un microballon expansible (16).

5. Dispositif de transfert de chaleur selon la revendication 3, dans lequel ledit élément d'amélioration de mélange (74) comprend un ballon à micro-anneau expansible (16).

6. Dispositif de transfert de chaleur selon la revendication 3, dans lequel ledit élément d'amélioration de mélange (74) comprend plusieurs saillies échelonnées axialement et se chevauchant circonférentiellement (54) situées le long de ladite surface extérieure.

7. Dispositif de transfert de chaleur selon la revendication 3, dans lequel ledit élément d'amélioration de mélange (74) comprend un fil détecteur de température (124) enroulé hélicoïdalement autour de ladite surface extérieure et ayant un capteur de température (128) en contact thermique avec ledit élément de transfert de chaleur (14) pour une commande de rétroaction dudit élément de transfert de chaleur (14).

8. Dispositif de transfert de chaleur selon la revendication 7, dans lequel ledit capteur de température (128) est un élément du groupe se composant d'un thermocouple et d'une thermistance.

9. Dispositif de transfert de chaleur selon la revendication 3, dans lequel ledit cathéter (12) comprend un axe longitudinal et ledit élément d'amélioration de mélange (74) comprend un ventilateur (164) apte à tourner autour d'un axe coaxial au dit axe longitudinal dudit cathéter.

10. Dispositif de transfert de chaleur selon la revendication 9, comprenant en outre un mécanisme d'entraînement (166) apte à faire tourner ledit ventilateur (164).

11. Dispositif de transfert de chaleur selon l'une quelconque des revendications précédentes, dans lequel le cathéter (20) comprend :
une lumière d'entrée (322, 354, 363, 604) ; et
une lumière de sortie (314, 320, 361, 365, 520, 606), la lumière de sortie étant couplée à la lumière d'entrée afin de transférer un fluide de travail entre celles-ci, l'une de la lumière d'entrée et de la lumière de sortie ayant une forme hélicoïdale,
dans lequel la lumière d'entrée et la lumière de sortie sont constituées d'un matériau flexible,
le matériau flexible étant un matériau capable de gonfler.

12. Dispositif de transfert de chaleur selon la revendication 11, comprenant en outre plusieurs lumières de sortie.

13. Dispositif de transfert de chaleur selon la revendication 12, dans lequel chaque lumière de sortie encercle hélicoïdalement la lumière d'entrée.

14. Dispositif de transfert de chaleur selon l'une quelconque des revendications 11 à 13, dans lequel le fluide de travail est salin.

15. Dispositif de transfert de chaleur selon l'une quelconque des revendications 11 à 14, comprenant en outre un fil (124) disposé au moins partiellement à l'intérieur de la lumière d'entrée ou de la lumière de sortie.

16. Dispositif de transfert de chaleur selon l'une quelconque des revendications 11 à 15, dans lequel une longueur de la lumière d'entrée est entre environ 5 cm et 30 cm.

17. Dispositif de transfert de chaleur selon l'une quelconque des revendications 11 à 16, comprenant en outre une alimentation de fluide de travail comprenant une pompe, et dans lequel la pompe fait circuler le fluide de travail.

18. Dispositif de transfert de chaleur selon la revendication 17, dans lequel l'alimentation de fluide de travail est configurée pour produire un fluide de travail pressurisé à une température entre environ -3°C et 36°C et à une pression inférieure à 5 atmosphères.

19. Dispositif de transfert de chaleur selon l'une quelconque des revendications 11 à 18, dans lequel la lumière de sortie encercle hélicoïdalement la lumière d'entrée.

20. Dispositif de transfert de chaleur la revendication 19, dans lequel la lumière de sortie comprend un revêtement ou traitement de surface pour empêcher la formation de caillot.
